(19) Europäisches Patentamt
European Patent Office
Office européen des brevets

(11) EP 0 544 592 B1

(12) **FASCICULE DE BREVET EUROPEEN**

(45) Date de publication et mention de la délivrance du brevet: **03.02.1999 Bulletin 1999/05**

(21) Numéro de dépôt: **92403180.0**

(22) Date de dépôt: **25.11.1992**

(51) Int. Cl.[6]: **C08B 37/10**, C12P 19/04, A61K 31/725 // (C12P19/04, C12R1:19)

# (54) Héparosanes-N,O-sulfates de haute masse moléculaire, leur procédé de préparation et les compositions pharmaceutiques qui les contiennent

Hochmolekulare, N,O-sulfatierte Heparosane; Verfahren zu deren Herstellung und diese enthaltende Arzneimittel

High molecular weight N,O-sulfated heparosans, process for producing the same and pharmaceutical compositions containing them

(84) Etats contractants désignés:
**AT BE CH DE DK ES FR GB GR IE IT LI LU MC NL PT SE**

(30) Priorité: **28.11.1991 FR 9114725**

(43) Date de publication de la demande: **02.06.1993 Bulletin 1993/22**

(73) Titulaire: **SANOFI**
**75008 Paris (FR)**

(72) Inventeurs:
- **Lormeau, Jean-Claude**
  **F-94270 Kremlin Bicetre (FR)**
- **Chevallier, Bruno**
  **F-94800 Villejuif (FR)**
- **Salome, Marc Louis Victor**
  **F-31320 Castanet-Tolosan (FR)**

(74) Mandataire: **Le Guen, Gérard et al**
**CABINET LAVOIX**
**2, place d'Estienne d'Orves**
**75441 Paris Cédex 09 (FR)**

(56) Documents cités:
**EP-A- 0 333 243** **EP-A- 0 489 647**
**WO-A-92/17507** **FR-A- 2 584 728**

- **BIOCHEMICAL JOURNAL vol. 275, 1 Avril 1991, GB pages 151 - 158 M. KUSCHE ET AL. 'Biosynthesis of heparin; Use of Escherichia coli K5 capsular polysaccharide as a model substrate in enzymic polymer-modification reactions'**
- **GLYCOCONJUGATE vol. 4, 1987, pages 179 - 189 J. RIESENFELD ET AL. 'Biosynthesis of heparin; Effect of detergent on the microsomal polymerization and polymer modification processes'**

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).

EP 0 544 592 B1

## Description

La présente invention a pour objet de nouveaux héparosanes-N,O-sulfatés de haute masse moléculaire, les compositions d'héparosanes-N,O-sulfatés contenant ces nouveaux héparosanes-N,O-sulfatés, et les compositions pharmaceutiques ayant comme principe actif les nouveaux héparosanes-N,O-sulfatés de haute masse moléculaire.

Il est connu que les glycosaminoglycanes sont des produits susceptibles d'être obtenus par extraction des tissus animaux. Certains de ces glycosaminoglycanes possèdent des propriétés anticoagulantes et antithrombotiques très intéressantes. Des produits typiques de cette famille sont l'héparine, ses fragments et leurs dérivés, ainsi que l'héparane-sulfate et le dermatane-sulfate, qui ont cependant le désavantage, dû à leur origine, d'être très coûteux.

En particulier, il est connu que le dermatane-sulfate est une famille de polymères de degré de polymérisation variable, formés d'unités répétées constituées d'un groupe acide uronique (iduronyle ou glucuronyle) et d'un groupe acétylsulfo-4-galactosaminyle (H.W. Stuhlsatz "The Methodology of Connective Tissue Research", (1976), 137-146). Le dermatane-sulfate naturel a une masse moléculaire comprise entre $2.10^4$ et $4.10^4$ Da. Ce produit est particulièrement intéressant comme anticoagulant et antithrombotique (F. Fernandez et al, British Journal of Haematology, (1986), 64, 309-317).

Il est d'autre part connu (I. Björk et U. Lindahl, "Molecular and Cellular Biochemistry", (1982), Dr. W. Junk Publishers - Pays-Bas) que la coagulation sanguine est un phénomène physiologique complexe, dont le mécanisme peut être schématisé et résumé de la façon suivante :

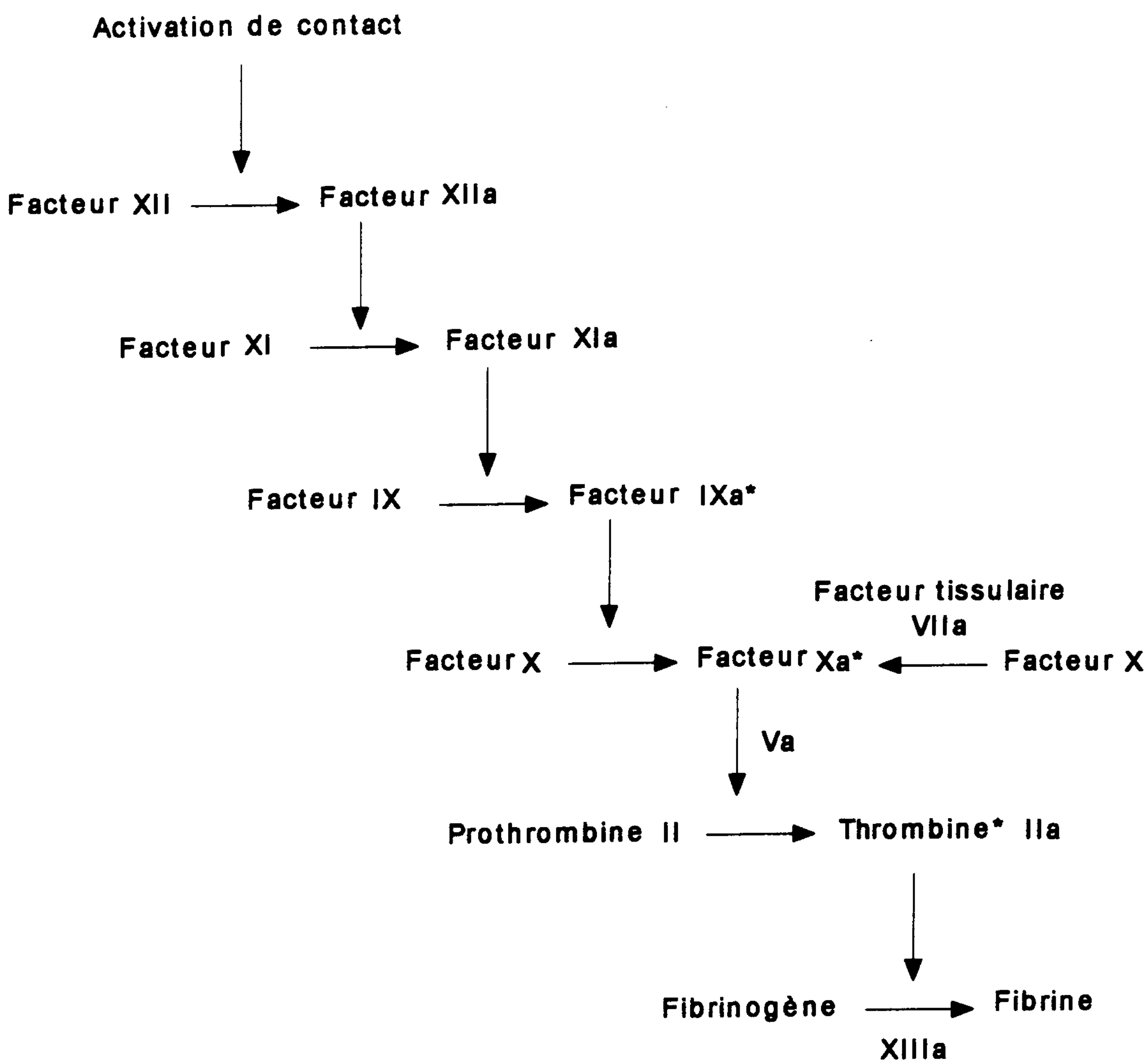


Certains stimuli, tels que l'activation de contact et les facteurs tissulaires, déclenchent l'activation successive d'une série de facteurs de coagulation présents dans le plasma sanguin, ceux-ci étant repérés par des chiffres romains

sur le schéma ci-dessus et la présence de l'indice a désignant la forme activée (indice a présent) ou non activée (indice a absent) d'un facteur de coagulation donné.

Quelle que soit la nature du stimulus, les étapes finales sont identiques : le facteur Xa active le facteur II (également appelé prothrombine), lequel sous sa forme activée (facteur IIa également appelé thrombine) provoque la protéolyse partielle du fibrinogène soluble avec libération de la fibrine insoluble, constituant principal du caillot sanguin.

Dans les conditions physiologiques normales, des protéines régulatrices telles que l'antithrombine III (ATIII) et le cofacteur II de l'héparine (HCII), sont également présentes dans le plasma.

L'antithrombine III exerce une activité inhibitrice sur l'ensemble des facteurs de coagulation repérés par un astérisque (*) dans le schéma ci-dessus. Cette inhibition est amplifiée de façon très forte en présence d'héparine ou d'oligosaccharides de synthèse de type héparinique (D.H. Atha et al, Biochemistry, (1985), 24, 6723-6729).

Le cofacteur II de l'héparine n'exerce une activité inhibitrice que sur le facteur IIa (thrombine), lequel catalyse la dernière étape de la coagulation. Cette activité est amplifiée de façon importante en présence d'héparine ou de dermatane-sulfate (D.M. Tollefsen, J. Biol. Chem, (1983), 258, 6713-6716).

L'inhibition du facteur Xa ou du facteur IIa constitue un moyen privilégié pour obtenir une activité anticoagulante et antithrombotique puisque ces deux facteurs interviennent dans les deux dernières étapes de la coagulation, lesquelles sont indépendantes du stimulus déclencheur.

Pour obtenir l'inhibition du facteur IIa seul, une possibilité particulièrement intéressante consiste à mettre à profit la spécificité du cofacteur II de l'héparine et à rechercher l'amplification de son activité inhibitrice. Le dermatane-sulfate est le produit connu possédant l'activité amplificatrice de ce type, la plus puissante.

Il est également connu que la constitution de la chaîne héparinique principale se fait en deux étapes. Dans un premier temps, l'héparine est biosynthétisée à partir d'un protéoglycane précurseur, dont la partie polysaccharidique est constituée par une famille de polymères de degré de polymérisation variable formés d'unités disaccharidiques répétées β-D-glucuronyl-1,4-α-N-acétyl-D-glucosaminyl-(1,4). Cette partie polysaccharidique est habituellement appelée N-acétylhéparosane (J. Navia, Anal. Biochem., (1983), 135, 134-140). Cette première étape de biosynthèse est le seul moment où l'on peut vraiment parler d'un "motif disaccharidique" car la seconde étape de la biosynthèse va modifier ce squelette simple d'une manière profonde ("L'héparine, fabrication, structure, propriétés, analyses", J.P. Duclos, (1984) pp. 81-83, Masson Ed.- France).

En effet, l'héparine naturelle, résultat de la biosynthèse, est un polysaccharide constitué par des molécules d'acide glucuronique et d'acide iduronique (acides uroniques), éventuellement sulfatées en position 2, associées à des molécules de glucosamine, éventuellement sulfatées en position 6 et sulfatées ou acétylées sur l'amine en position 2.

Il est aussi connu que certaines bactéries de l'espèce Escherichia coli produisent un polysaccharide capsulaire, habituellement appelé antigène K5, qui est une famille de polymères constitués d'unités répétées β-D-glucuronyl-1,4-α-N-acétyl-D-glucosaminyl-(1,4), (W.F. Vann et al, Eur. J. Biochem, (1981), 116, 359-364).

Ce polysaccharide, de même nature chimique que la partie polysaccharidique du protéoglycane précurseur de l'héparine, sera appelé ici N-acétylhéparosane. Ce produit possède une masse moléculaire comprise entre $10^5$ et $2,0.10^6$ Da, et au niveau des unités "acide uronique", une structure très régulière composée uniquement d'acide D-glucuronique (W.F. Vann et al, Eur. J. Biochem., (1981), 116, 359-364, et demande de brevet EP-A-0 333 243).

La demande de brevet EP-A-0 333 243 décrit le polysaccharide K5 O-sulfaté (N-acétylhéparosane O-sulfaté) ainsi que certains de ses fragments, composés respectivement de 4, 6 ou 8 unités "sucres", également O-sulfatés. Ces produits ont une activité antiangiogénique et antitumorale, avec un rapport favorable de ces activités par rapport aux propriétés anticoagulantes. Ce document décrit également des fragments de N-acétylhéparosane composés respectivement de 4, 6, 8 ou 10 unités "sucres", et aussi des N-acétylhéparosanes O-sulfatés de haute masse moléculaire. Ces derniers produits, soumis à une dépolymérisation drastique, selon la méthode décrite par W.F. Vann et al dans Eur. J. Biochem., (1981), 116, 359-364, donnent de petits fragments de N-acétylhéparosanes O-sulfatés, constitués de 8, 6 ou 4 unités "sucre" tels que décrits dans le brevet EP-A-0 333 243.

La demande de brevet EP-A-0 333 243 décrit aussi la préparation d'un pentasaccharide ayant la structure N-acétylhéparosane-O-sulfaté, par synthèse chimique totale.

Il est également connu que le polysaccharide K5, N-désacétylé et N-sulfaté, peut-être utilisé comme substrat pour les enzymes qui catalysent les dernières réactions de modification du polymère, à savoir la hexuronosyl-C5-épimérisation et la O-sulfatation dans la biosynthèse de l'héparine (M. Kusche et al, Biochem. J. (1991), 275, 151-158). M. Kusche et al décrivent la préparation du polysaccharide K5 N-désacétylé-N-sulfaté par N-désacétylation à l'aide de l'hydrazine/sulfate d'hydrazine et N-sulfatation par traitement du produit N-désacétylé avec le complexe triméthylamine-trioxyde de soufre.

Il est enfin connu que par 5-épimérisation partielle et O-sulfatation du polysaccharide K5 C5-épimérisé, N-désacétylé et N-sulfaté, on obtient des produits qui ont une activité anti-Xa et une activité anti-IIa via HCII qui se situe entre celle typique de l'héparine de muqueuse et celle de l'héparane sulfate (B. Casu et al, International Symposium on Heparin and related polysaccharides, UPPSALA, 2-6 septembre 1991, Abstract N°12). Tous ces produits, comme l'héparine, ont dans leurs chaînes aussi bien des structures glucuroniques que des structures iduroniques. Des dérivés

du polysaccharide K5 C5-épimérisés N-désacétylés et N-sulfatés comportant dans leurs chaînes aussi bien des structures glucuroniques que des structures iduroniques sont aussi décrits par M. Kusche et al, Biochem. J. (1991), 275, 151-158.

J. Riesenfeld et al (Glycoconjugate (1987), 4, 179-189) décrivent, lors d'une étude destinée à élucider la biosynthèse de l'héparine, la sulfatation d'un N-acétylhéparosane par une enzyme, sans mentionner la structure exacte des produits obtenus.

Par ailleurs, on sait que le brevet français N° 2.584.728 du 10.11.87 (correspondant à la demande FR-85.10787) décrit un procédé de sulfatation de glycosaminoglycanes, et notamment de l'héparine, permettant d'introduire des groupes esters sulfuriques (groupe $-SO_3^-$) à la place de groupes hydroxyles (-OH) primaires sur les unités saccharidiques du glycosaminoglycane de départ, sans modifier son degré de polymérisation ni son homogénéité.

On a maintenant trouvé que par O-sulfatation du polysaccharide K5 N-désacétylé et N-sulfaté, on obtient un héparosane-N,O-sulfaté de haute masse moléculaire qui possède une bonne activité sur la coagulation, ladite activité se manifestant via l'héparine cofacteur II et, ce qui est surprenant, via l'antithrombine III. Plus particulièrement, on a trouvé de façon inattendue, que la 5-épimérisation partielle n'est pas nécessaire et que les héparosanes-N,O-sulfatés, contenant dans leurs chaînes, à côté des unités de glucosamine, uniquement des unités glucuroniques, peuvent être utilisés comme principes actifs de médicaments anticoagulants et antithrombotiques.

Les héparosanes-N,O-sulfatés de haute masse moléculaire, objet de la présente invention, se distinguent donc des autres produits déjà décrits dans la littérature, par leur structure originale, notamment caractérisée par un degré de sulfatation élevé (sulfatation aussi sur le groupe amine de la glucosamine), par l'absence d'acide iduronique et par leurs propriétés pharmacologiques inattendues. Ils possèdent une activité anticoagulante vis à vis de l'héparine cofacteur II (HCII) plus forte que celle du dermatane-sulfate. En effet, leurs activités pharmacologiques sont comparables à celles des glycosaminoglycanes couramment utilisés en thérapeutique, notamment celles de l'héparine, et peuvent être utiles pour la régulation de la coagulation. Les produits de l'invention trouvent plus particulièrement leurs applications comme antithrombotiques.

L'expression "de haute masse moléculaire" telle qu'utilisée dans la présente description pour le polysaccharide K5 et pour ses dérivés N-désacétylés, N-désacétylés et N-sulfatés ainsi que pour les héparosanes-N,O-sulfatés objet de la présente invention se réfère à une masse moléculaire supérieure à $1,5.10^4$ Da, à savoir au delà de la limite dans laquelle ladite masse moléculaire est déterminée avec une certaine précision selon les méthodes analytiques courantes. Lorsque l'on se réfère au polysaccharide K5 naturel et à ses dérivés, il est entendu que leur masse moléculaire est celle indiquée dans les références W.F. Vann et al (Eur. J. Biochem, (1981), 116, 359-364) et EP-A-0 333 243 ci-dessus sauf si diversement spécifiés. Toutefois, la même expression inclut également des produits ayant une masse moléculaire inférieure à celle du polysaccharide K5 naturel, obtenus par fractionnement et/ou fragmentation et formés par des chaînes de masse moléculaire comprise entre $1,5.10^4$ et $10^5$ Da. En définitive, l'expression "haute masse moléculaire" s'applique à tout produit de type polysaccharide K5, y compris les fractions, éventuellement N-désacétylés et N-sulfatés ou à tout héparosane-N,O-sulfaté, y compris ses fractions, ayant une masse moléculaire comprise entre $1,5.10^4$ et $4,0.10^6$ Da., à l'exclusion des héparosanes-N,O-sulfatés de masse moleculaire egale à $1,5.10^4$ Da.

La présente invention a pour objet des héparosanes-N,O-sulfatés constitués par des chaînes ou par un mélange de chaînes de masse moléculaire entre $1,5.10^4$ et $4,0.10^6$ Da, ayant la structure disaccharidique répétée de formule I :

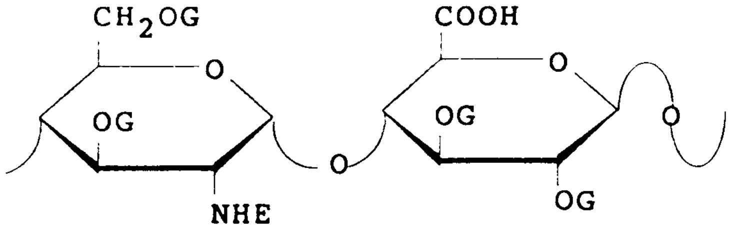

(I)

dans laquelle,

- R représente, dans 0 à 80 % des unités disaccharidiques desdits héparosanes-N,O-sulfatés, un groupe acétyle, et dans les unités disaccharidiques restantes, un groupe sulfate et éventuellement un atome d'hydrogène,
- G représente un atome d'hydrogène et un groupe sulfate,
  et ayant un degré de sulfatation exprimé comme rapport sulfate/carboxyle de 1,5 à 3,0,
  à l'exclusion des héparosanes N,O-sulfatés constitués par des chaînes ayant une masse moléculaire de $1,5.10^4$ Da,

et les sels pharmaceutiquement acceptables desdits héparosanes-N,O-sulfatés.

L'invention concerne aussi une composition d'héparosane-N,O-sulfaté contenant au moins 70 % en masse d'un héparosane-N,O-sulfaté décrit ci-dessus et objet de la présente invention.

Les héparosanes-N,O-sulfatés objet de la présente invention peuvent être constitués de chaînes polysaccharidiques identiques de masse moléculaire bien définie, cette masse moléculaire étant située dans l'intervalle $1,5.10^4$ et $4,0.10^6$ Da. Ils peuvent aussi être constitués par un mélange de chaînes de masses moléculaires variables, ces masses moléculaires étant comprises entre $1,5.10^4$ et $4,0.10^6$ Da. La dispersion des masses moléculaires de ces chaînes peut-être plus ou moins importante. En effet, les héparosanes-N,O-sulfatés, objet de la présente invention peuvent être constitués par des chaînes ayant entres elles une différence de masse moléculaire d'au maximum environ $3,9.10^6$ Da, ou au contraire, seulement par des chaînes ayant entre elles une différence de masse moléculaire d'environ 300 Da, ce qui correspond à une unité de structure uronique (acide D-glucuronique ou ses dérivés) ou de structure glucosamine, ou encore des chaînes ayant entre elles une différence de masse moléculaire inférieure à 300 Da dans le cas où les héparosanes-N,O-sulfatés résultent d'une fragmentation chimique. Il est aussi évident qu'en fonction de la constitution de chaque héparosane-N,O-sulfaté, la masse moléculaire des chaînes ayant, soit la masse moléculaire la plus faible, soit la masse moléculaire la plus haute, peut correspondre à n'importe quelle valeur comprise entre $1,5.10^4$ et $4,0.10^6$ Da.

L'expression "G représente un atome d'hydrogène et un groupe sulfate" utilisée ci-dessus, indique que G dans l'unité disaccharidique de formule I, représente pour certaines positions un atome d'hydrogène et dans les autres restantes, un groupe sulfate. De la même manière, E représente dans certaines unités disaccharidiques un groupe acétyle et dans le reste de ces unités, un groupe sulfate ou éventuellement un atome d'hydrogène. Les unités disaccharidiques des héparosanes-N,O-sulfatés, peuvent donc ne pas être toutes identiques.

La formule I représente une structure répétitive disaccharidique formée par une unité glucosamine et une unité acide D-glucuronique. Lesdites unités peuvent être inversées, plus particulièrement, si l'on considère que la structure disaccharidique de formule I est répétée n fois et que l'unité non réductrice des chaînes peut-être indifféremment soit une unité glucosamine, telle que représentée dans la formule I avec un groupe hydroxy en position 4 cette unité de glucosamine étant sulfatée ou non, soit un acide D-glucuronique contenant éventuellement une double liaison en position C4-C5 et étant sulfaté ou non. L'unité réductrice peut-être indifféremment soit un acide D-glucuronique, tel que représenté dans la formule I, substitué par un hydrogène sur l'oxygène anomérique, soit une glucosamine. Les composés préférés de l'invention sont constitués de chaînes dont les extrémités réductrices et non réductrices sont des unités uroniques sulfatées ou non, de la glucosamine sulfatée ou non ou de la N-acétylglucosamine sulfatée ou non.

Comme dans le cas de l'héparine et sauf indication contraire, on entend par les termes "polysaccharide K5" et "héparosane-N,O-sulfaté" référés aux produits, les produits sous forme de sel de sodium.

Selon un autre de ses aspects, la présente invention a pour objet un procédé de préparation d'héparosanes-N,O-sulfatés de haute masse moléculaire caractérisé en ce que :

(i) soit on soumet un produit de départ constitué par un polysaccharide K5 N-désacétylé, N-sulfaté, ou par une de ses fractions, à une O-sulfatation,

(ii) soit on soumet un produit de départ constitué par un polysaccharide K5 N-désacétylé, ou par une de ses fractions, à une N,O-sulfatation partielle suivie éventuellement d'une N-sulfatation totale, puis l'on isole le produit ainsi obtenu et on le transforme éventuellement en un sel pharmaceutiquement acceptable.

On utilise, comme produits de départ, des produits qui ont une masse moléculaire comprise entre $1,0.10^4$ et $2,0.10^6$ Da, pour préparer des héparosanes-N,O-sulfatés objet de la présente invention. A partir des produits ayant une masse moléculaire d'environ $2,0.10^6$ Da, on obtient des héparosanes-N,O-sulfaté, ayant une masse moléculaire d'environ $4,0.10^6$ Da. La différence de masse indiquée entre les produits de départ et les héparosanes-N,O-sulfatés, est due à la N,O-sulfatation.

La présente invention a aussi plus particulièrement pour objet un procédé de préparation d'une composition contenant 70 % à 100 % d'un héparosane-N,O-sulfaté de haute masse moléculaire, objet de la présente invention, caractérisé en ce qu'il comprend la séquence des étapes suivantes :

- étape a : culture d'une souche d'*Escherichia coli* (K5), pour former un N-acétylhéparosane (polysaccharide K5) de haute masse moléculaire,

- étape b : isolement et purification du N-acétylhéparosane formé pour obtenir une composition contenant 70 % à 100 % d'un N-acétylhéparosane constitué par un mélange de chaînes de masse moléculaire entre $1,0.10^4$ et $2,0.10^6$ Da, caractérisées par une structure disaccharidique répétée de formule II :

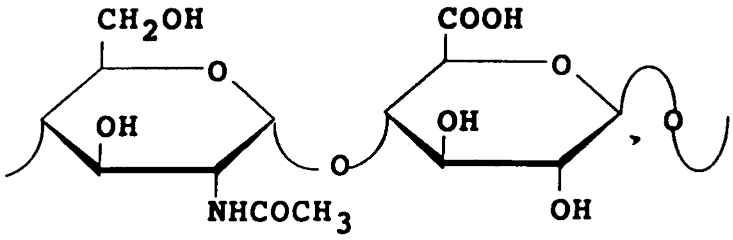

(II)

- étape c : désacétylation de cette composition de N-acétylhéparosane pour obtenir une composition contenant 70 % à 100 % d'un héparosane constitué par un mélange de chaînes de masse moléculaire entre $1,0.10^4$ et $2,0.10^6$ Da, caractérisées par une structure disaccharidique répétée de formule III :

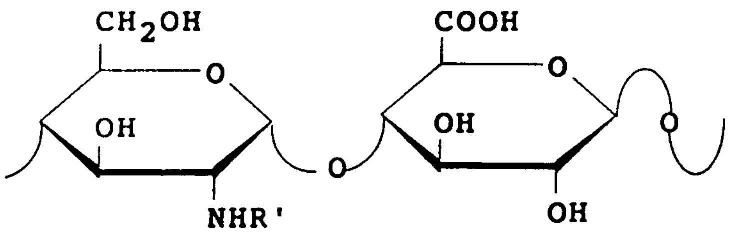

(III)

dans laquelle R' représente, dans 0 à 80 % des unités disaccharidiques, un groupe acétyle, et dans les unités disaccharidiques restantes un atome d'hydrogène,

- étape d :

  - soit une N-sulfatation totale ou partielle pour obtenir une composition contenant 70 % à 100 % d'un héparosane N-sulfaté constitué par un mélange de chaînes de masse moléculaire entre $1,0.10^4$ et $2,0.10^6$ Da caractérisées par une structure disaccharidique répétée de formule IV :

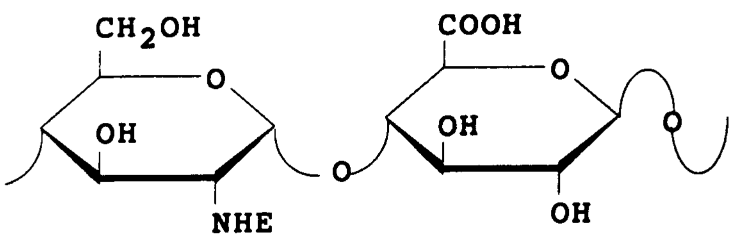

(IV)

  dans laquelle E représente dans 0 à 80 % des unités disaccharidiques un groupe acétyle et dans les unités disaccharidiques restantes un groupe sulfate et éventuellement un atome d'hydrogène,
  cette étape de N-sulfatation partielle ou totale étant suivie d'une étape de O-sulfatation partielle ou totale,
  - soit une N,O-sulfatation partielle de cette composition d'héparosane,
  - soit une N,O-sulfatation partielle de cette composition d'héparosane suivie d'une étape de N-sulfatation totale,

et comporte éventuellement une ou plusieurs étapes de fractionnement des masses moléculaires effectuées à la fin des étapes a, b, c ou d.

Comme produit de départ, on peut utiliser un polysaccharide K5 N-désacétylé, N-sulfaté tel que celui décrit par M. Kusche et al (Biochem J. (1991), 275, 151-158).

Un autre produit de départ avantageux, est un polysaccharide K5 tel que décrit dans EP-A-0 333 243.

Un produit de départ particulièrement avantageux est un polysaccharide K5 obtenu par culture d'Escherichia

coli SEBR 3282. Cette souche est une souche dérivée de la souche Bi 8337-41 (O10: K5:H4) ATCC 23506 (décrite notamment par D.S. Gupta et al FEMS Microbiology Letters, (1982), 14, 75-78 et W. Vann Eur. J. Biochem., (1981), 116, 359-364).

La souche Escherichia coli SEBR 3282 répond positivement au test de typage par le phage K5 spécifique, selon la méthode de B. Kaiser et al (J. Clin. Microbiol., ( 1984), 19, 2, 264-266). Il s'agit donc bien d'une souche Escherichia coli (K5). Cette souche a été déposée auprès de la CNCM de l'Institut Pasteur, Paris, France, sous le N° I-1013. On peut aussi utiliser un mutant, soit spontané soit induit de cette souche, comme aussi d'autres souches d'Escherichia coli (K5) appropriées par exemple la souche Bi 626-42 (012:K5(L):NM) ATCC 23508.

Pour la préparation d'un N-acétylhéparosane de haute masse moléculaire utilisé comme matière première pour l'hémisynthèse chimique des produits de l'invention, un milieu de culture riche en glycérol est utilisé de préférence au glucose.

La culture de la souche d'Escherichia coli (K5) est continuée de préférence au moins deux heures après l'arrêt de la croissance de la biomasse (environ 25-35 heures d'âge en fermenteur).

A l'étape b, l'isolement et la purification du N-acétylhéparosane, pour obtenir une composition contenant 70 % à 100 % d'un N-acétylhéparosane constitué par un mélange de chaînes de masse moléculaire entre $1,0.10^4$ et $2,0.10^6$ Da, caractérisées par une structure disaccharidique répétée de formule II, sont réalisés par un procédé comprenant au moins une étape de précipitation et une étape de chromatographie d'échange d'ions. Cette dernière étape est réalisée en utilisant de préférence une colonne Q-Sepharose ou une colonne équivalente. La précipitation est effectuée par un solvant organique approprié, et notamment un alcool, de préférence l'éthanol. Lors de ce procédé, le N-acétylhéparosane peut-être sous forme de sel, de préférence sous forme de sel de sodium.

A titre d'exemple, on peut schématiser le procédé préféré d'isolement et de purification comme suit :

- étape $a_1$ : Précipitation par l'éthanol,
- étape $b_1$ : Dialyse,
- étape $c_1$ : Précipitation par l'éthanol, puis déshydratation et séchage,
- étape $d_1$ : Purification par chromatographie d'échange d'anions,
- étape $e_1$ : Précipitation par l'éthanol de l'éluat, obtenu par l'étape $d_1$, déshydratation, séchage et broyage.

Concernant les étapes $a_1$, $b_1$, $c_1$, l'ordre avec lequel elles sont effectuées importe peu. Une des étapes $a_1$ ou $c_1$ peut être supprimée.

A l'étape $e_1$, la précipitation par l'éthanol n'est pas indispensable. Il est possible d'isoler le N-acétylhéparosane par d'autres méthodes comme par exemple par évaporation sous vide de l'éluat, obtenu par l'étape $d_1$.

L'isolement et la purification du N-acétylhéparosane, pour obtenir une composition contenant 70 % à 100 % d'un N-acétylhéparosane constitué par un mélange de chaînes de masse moléculaire comprise entre $1,5.10^4$ et $2,0.10^6$ Da environ peut aussi être effectuée de la manière suivante :

- étape $a'_1$ : Dialyse,
- étape $b'_1$ : Purification en milieu acide, élimination des impuretés insolubles dans des solutions aqueuses de pH 3,5 et pH 1,8,
- étape $c'_1$ : Précipitation par l'éthanol, puis déshydratation et séchage,
- étape $d'_1$ : Hydrolyse alcaline et dialyse,
- étape $e'_1$ : Purification par chromatographie d'échange d'anions,
- étape $f'_1$ : Purification par chromatographie d'exclusion.

Ce procédé d'isolement et de purification est aussi un procédé préféré de l'invention.

L'hydrolyse alcaline est effectuée avec une solution de NaOH, à une température comprise entre 30°C et 80°C.

En appliquant les procédés d'isolement et de purification, on peut utiliser comme produit de départ, soit la suspension obtenue à la fin de la culture et dans ce cas une filtration préalable est nécessaire, soit un produit qui est déjà soumis à une purification préliminaire effectuée selon un procédé qui comporte les étapes suivantes :

- étape $a''_1$ : Centrifugation de la suspension obtenue à la fin de la culture,
- étape $b''_1$ : Mise en contact du surnageant avec une solution alcaline,
- étape $c''_1$ : Préfiltration,
- étape $d''_1$ : Concentration sur membrane de seuil de coupure déterminé, et éventuellement,
- étape $e''_1$ : Dialyse.

Comme solution alcaline, on peut ici aussi utiliser une solution de NaOH.

De manière préférentielle, on procède à une purification préliminaire du produit obtenu à la fin de la culture, selon la méthode décrite ci-dessus.

Les deux extrémités réductrice et non réductrice des N-acétylhéparosanes de formule II tels qu'obtenus selon le procédé décrit ci-dessus et en utilisant la souche d'Escherichia coli SEBR 3282 ou une autre souche appropriée comme indiqué précédemment, sont des unités uroniques ou de la N-acétylglucosamine.

Dans la majorité des chaînes, l'extrémité non réductrice est une unité uronique de formule (a) :

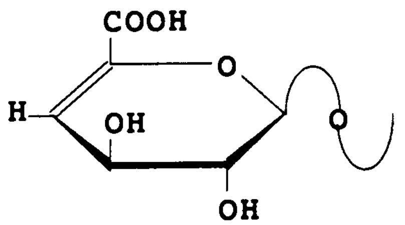

(a)

L'enrichissement éventuel de la composition obtenue en N-acétylhéparosane, constituée par un mélange de chaînes de masse moléculaire entre $1,5.10^4$ et $2,0.10^6$ Da, ayant une structure disaccharidique répétée de formule II, peut-être effectué à des degrés divers et notamment jusqu'à l'isolement dudit N-acétylhéparosane. L'enrichissement est réalisé par la mise en oeuvre de techniques classiques de fractionnement des masses moléculaires telles que la chromatographie de perméation de gel et l'ultrafiltration (A.A. Horner, Biochem. J., (1989), 262, 953-958 ; G. Pyler et al, J. Biol. Chem. (1988), 263, 11, 5197-5201 et U. Lindahl et al, J. Biol. Chem., (1984), 259, 20, 12368-12376). On peut aussi utiliser la méthode de fractionnement éthanolique (Demande de Brevet EP-A-0 287 477). Cette dernière méthode de fractionnement est particulièrement appréciée parmi d'autres méthodes envisageables.

A l'étape c, la désacétylation partielle des compositions contenant 70 % à 100 % de N-acétylhéparosane, qui conduit à l'obtention des compositions contenant 70 % à 100 % d'un héparosane de haute masse moléculaire constitué par un mélange de chaînes de masse moléculaire comprise entre $1,0.10^4$ et $2,0.10^6$ Da, caractérisées par une structure disaccharidique répétée de formule III, indiquée ci-dessus, est réalisée par un traitement avec un agent désacétylant.

Comme agents désacétylants on peut mentionner le pentasulfure de phosphore, le fluoroborate de triéthyloxonium, la soude ou l'hydrazine, ces deux derniers agents étant particulièrement appréciés. On peut aussi utiliser les acides minéraux forts, tels que l'acide chlorhydrique, l'acide sulfurique etc.. La durée de la réaction dépend des conditions opératoires choisies et notamment de la température et de la concentration de l'agent désacétylant dans le milieu réactionnel.

L'enrichissement de la composition de l'héparosane en héparosane constitué d'un mélange de chaînes de masse moléculaire comprise entre $1,0.10^4$ et $2,0.10^6$ Da, caractérisées par une structure disaccharidique répétée de formule III, est réalisé par la mise en oeuvre de techniques classiques de fractionnement des masses moléculaires mentionnées ci-dessus (chromatographie de gel perméation, ultrafiltration et fractionnement par des solvants organiques miscibles à l'eau et notamment fractionnement éthanolique). Dans ce cas, il est possible d'obtenir des compositions contenant 90 % à 100 % en masse d'un héparosane constitué par un mélange de masse moléculaire entre $1,0.10^4$ et $2,0.10^6$ Da, ayant la structure disaccharidique répétée de formule III.

Selon la première variante de l'étape d (N-sulfatation partielle ou totale suivie d'une O-sulfatation partielle ou totale), la N-sulfatation est effectuée selon des méthodes connues (M. Kusche et al, Biochem. J. (1991) 275, 151-158). Plus précisément, la N-sulfatation est réalisée à l'aide d'un complexe de trioxyde de soufre avec une base organique telle que la triméthylamine, la triéthylamine ou la pyridine. Elle peut aussi être effectuée par l'acide chlorosulfonique en solution dans la pyridine. On utilise avantageusement le complexe trioxyde de soufre ($SO_3$) avec la triméthylamine, et la réaction de la N-sulfatation est réalisée à une température comprise entre 20°C et 80°C en milieu aqueux alcalin. A la fin de la réaction de N-sulfatation, le produit ainsi obtenu est précipité par addition d'une quantité adéquate d'éthanol. Le précipité formé est repris avec de l'eau ultra-purifiée, dialysé contre celle-ci, lyophilisé et séché. Ce processus de purification est donné à titre d'exemple et n'exclut pas des procédés équivalents. Les étapes de purification peuvent être répétées plusieurs fois.

Par la suite, et selon le procédé de l'invention, avant l'étape de la O-sulfatation, l'héparosane N-sulfaté est transformé de préférence en un sel de base organique ou en un sel d'ammonium quaternaire. Pour la formation du sel d'ammonium quaternaire on utilise, de manière préférentielle, l'hydroxyde de tetrabutylammonium.

La réaction de O-sulfatation est réalisée dans le formamide ou un autre solvant, chimiquement équivalent, à l'aide par exemple d'un complexe de trioxyde de soufre avec une base organique telle que la triméthylamine, la triéthylamine ou la pyridine ou encore à l'aide de l'acide chlorosulfonique en présence de pyridine. On utilise de préférence un complexe trioxyde de soufre-pyridine. La réaction de O-sulfatation est, en général, effectuée à une température comprise entre 10°C et 50°C.

L'héparosane-N,O-sulfaté est ensuite précipité par addition dans le milieu réactionnel de chlorure de sodium jusqu'à obtention d'une solution de 0,3 M à 0,5 M en NaCl, puis, d'une quantité adéquate d'éthanol. On procède ensuite à une purification de la composition de l'héparosane-N,O-sulfaté de la manière suivante : on reprend le précipité avec une solution de NaCl 0,3 M à 0,5 M, on reprécipite avec de l'éthanol, le précipité formé est isolé, repris avec de l'eau ultra-purifiée, dialysé contre celle-ci et éventuellement lyophilisé et séché.

Le processus de la purification ci-dessus mentionné est donné à titre d'exemple et n'exclut pas des procédés équivalents. La purification peut être effectuée une ou plusieurs fois.

L'enrichissement de la composition de l'héparosane-N,O-sulfaté en héparosane-N,O-sulfaté de masse moléculaire désirée, est réalisé par la mise en oeuvre des techniques classiques de fractionnement des masses moléculaires, déjà mentionnées. Il est intéressant d'effectuer cet enrichissement.

Selon la deuxième et la troisième variante de l'étape d du procédé (N,O-sulfatation partielle de la composition d'héparosane ou N,O-sulfatation partielle suivie d'une étape de N-sulfatation totale), avant l'étape de la N,O-sulfatation partielle, les héparosanes peuvent être transformés en un sel de base organique ou en un sel d'ammonium quaternaire. Pour la formation du sel d'ammonium quaternaire des héparosanes, on utilise de manière préférentielle l'hydroxyde de tetrabutylammonium.

L'étape de la N,O-sulfatation partielle réalisée selon le procédé décrit dans le brevet français N° 2 584 728 du 10.11.87 (correspondant à la demande FR-85.10787), est effectuée dans un solvant aprotique polaire, tel que le diméthylformamide, le diméthylsulfoxyde, l'hexaméthylphosphoramide ou l'acétonitrile ou un mélange de ces solvants, à l'aide par exemple d'un complexe de trioxyde de soufre avec une base organique, telle que la triméthylamine, la triéthylamine ou la pyridine. Elle peut aussi être effectuée par l'acide chlorosulfonique en solution dans la pyridine. On utilise de préférence le complexe trioxyde de soufre-pyridine.

Il est aussi possible d'utiliser d'autres agents de sulfatation, notamment ceux qui sont rapportés par E.E. Gilbert dans Chemical Review, (1962), 62, 549-589. La réaction de N,O-sulfatation est en général effectuée à une température comprise entre 0° et 100°C, de préférence entre 10°C et 50°C, pendant une durée comprise entre 6 h et 14 h.

Lors du procédé de la préparation, à la fin de la réaction de N,O-sulfatation partielle, la composition de l'héparosane-N,O-sulfaté contenant 70 % à 100 % d'un héparosane-N,O-sulfaté objet de la présente invention, est précipitée par addition de chlorure de sodium jusqu'à obtention d'une solution de 0,3 M à 0,5 M en chlorure de sodium, puis d'une quantité adéquate d'éthanol. Le précipité formé est repris dans une solution de chlorure de sodium 0,3 M à 0,5 M. Cette solution est ensuite neutralisée. Après addition d'une quantité adéquate d'éthanol, le précipité formé est isolé, repris avec de l'eau ultra-purifiée, dialysé contre celle-ci, lyophilisé et séché.

L'étape de la N,O-sulfatation, ainsi que les étapes de purification de la composition de l'héparosane-N,O-sulfaté décrites dans le paragraphe précédent, peuvent être répétées une ou plusieurs fois. Ici aussi, le processus de la purification est donné à titre d'exemple et n'exclut pas des procédés équivalents.

Cette étape de N,O-sulfatation est de préférence suivie d'une étape de N-sulfatation totale, réalisée en général dans un solvant aqueux, avantageusement de pH basique, avec un agent de sulfatation, tel qu'un complexe trioxyde de soufre avec une base organique, par exemple la triméthylamine, selon la technique de N-sulfatation mentionnée ci-dessus.

A la fin de l'étape de N-sulfatation totale, la composition de l'héparosane-N,O-sulfaté est précipitée après addition de chlorure de sodium, jusqu'à l'obtention d'une solution de 0,3 M à 0,5 M en chlorure de sodium, et d'éthanol. Le précipité formé est ensuite remis en solution dans une solution de chlorure de sodium 0,3 M à 0,5 M, reprécipité par l'éthanol, puis repris dans l'eau ultra-purifiée, dialysé contre celle-ci, lyophilisé et séché.

Comme il a déjà été mentionné, il est intéressant d'effectuer un enrichissement de la composition de l'héparosane-N,O-sulfaté en héparosane-N,O-sulfaté. Ceci est réalisé par la mise en oeuvre des techniques classiques de fractionnement des masses moléculaires.

Les héparosanes-N,O-sulfatés, objet de la présente invention et les compositions d'héparosane-N,O-sulfaté contenant au moins 70 % d'un nouvel héparosane-N,O-sulfaté, objet de la présente invention, sont avantageusement obtenus soit par un procédé comportant une réaction finale de N-sulfatation soit par un procédé comportant d'abord une N-sulfatation suivie d'une O-sulfatation partielle ou totale. E représente alors pour les structures répétées dans la formule (I), le groupe acétyle et le groupe sulfate.

On apprécie que les héparosanes-N,O-sulfatés objet de la présente inventions contiennent moins de 0,2 μmole/mg de groupe amino libre ($NH_2$).

Le groupe acétyle des unités disaccharidiques est de préférence présent à un taux inférieur ou égal à 60 °% et on apprécie que le degré de sulfatation, exprimé comme le rapport sulfate/carboxyle soit compris entre 1,5 et 3,0.

Les produits préférés de l'invention sont les héparosanes-N,O-sulfatés constitués par au moins 70 % en masse de chaînes de masse moléculaire comprise entre $1{,}0.10^5$ et $5{,}0.10^5$ Da, comme aussi les héparosanes-N,O-sulfatés constitués d'au moins 70 % en masse, de chaînes de masse moléculaire comprise entre $2,5.10^4$ et $2,5.10^5$ Da.

Comme autres produits préférés de l'invention, on peut citer les héparosanes-N,O-sulfatés constitués d'au moins 70 % en masse de chaînes de masse moléculaire comprise entre $2,0.10^4$ et $10^5$ Da.

Le procédé d'obtention des héparosanes-N,O-sulfatés décrit ci-dessus ainsi que les méthodes de purification permettent l'obtention des héparosanes-N,O-sulfatés sous forme de sel de sodium. A partir de ces sels, on peut obtenir, en appliquant les méthodes utilisées pour préparer les différents sels d'héparine ou d'héparine non salifiée ("L'héparine, fabrication, structure, propriétés, analyses", J.P. Duclos, (1984) pp. 81-83, Masson Ed. - France), soit d'autres sels d'héparosanes-N,O-sulfatés, soit des héparosanes-N,O-sulfatés non salifiés. Comme sels des héparosanes-N,O-sulfatés on entend tous les sels pharmaceutiquement acceptables. Ces sels sont obtenus par des méthodes classiques décrites notamment pour la préparation des sels de l'héparine (Brevet US 4 168 377).

Les héparosanes-N,O-sulfatés, objet de la présente invention, possèdent des propriétés pharmacologiques et biochimiques intéressantes et tout à fait surprenantes par rapport aux enseignements de l'art antérieur. Notamment, contrairement aux produits sulfatés de l'antigène K5 décrits dans le brevet EP-A-0 333 243, qui ont une activité antiangiogénique et antitumorale ou même encore une activité contre les virus enveloppés, avec un rapport favorable de ces activités par rapport aux propriétés anticoagulantes, les héparosanes-N,O-sulfatés de la présente invention possèdent une bonne activité régulatrice de la coagulation. Cette activité est très supérieure à celle du dermatane-sulfate sur les différents paramètres de la coagulation, et elle peut plutôt être comparée à celle de l'héparine.

Plus particulièrement, l'activité anti-IIa, ATIII ou HCII dépendante, de produits représentatifs de l'invention a été déterminée selon les méthodes décrites par D. Dupouy et al dans Thrombosis and Haemostasis, (1988), 60, 2, 236-239, pour le cofacteur II de l'héparine (HCII) et par M.L. Larsen et al dans Thrombosis Research, (1978), 13, 2, 285-288 pour l'antithrombine (ATIII).

Dans les deux cas, le test consiste à mesurer in vitro l'effet inhibiteur du produit étudié sur la thrombine purifiée (facteur IIa) en présence d'HCII ou ATIII purifiés dans le dosage de l'activité amidolytique de la thrombine, vis-à-vis d'un substrat chromogène. Comme il possède l'activité anti IIa HCII dépendante la plus forte, le dermatane-sulfate, préparé selon la méthode décrite par H.W. Stuhlsatz et al dans "The Methodology of Connective Tissue Research" (1976) 137-146, est utilisé comme produit de référence dans le test de mesure de cette activité, le résultat étant exprimé en mg de dermatane-sulfate (DS) équivalents en activité à 1 mg du produit étudié (mg DS équiv/mg).

L'activité anti-Xa (titre Yin-Wessler) desdits produits représentatifs de l'invention a été mesurée par la méthode décrite par E.T. Yin et al dans J. Lab. Clin. Med. (1973), 81, 2, 298-310, tandis que leur activité anticoagulante globale a été mesurée selon le test APTT décrit par R.R. Proctor et al dans Am. J. Clin. Path. (1961), 36, 212-219. Tous les produits testés ont montré une activité anti-IIa HCII dépendante nettement supérieure à celle du dermatane-sulfate. L'activité anti-IIa ATIII dépendante et le titre de Yin-Wessler, bien qu'inférieurs à ceux des héparines, se sont montrés supérieurs à ceux du dermatane-sulfate. Leur titre en APTT est d'environ 2 à environ 15 fois supérieur à celui du dermatane-sulfate, et peut atteindre 40 % de celui de l'héparine.

Les héparosanes-N,O-sulfatés de l'invention présentent donc une spécificité d'action et une activité anticoagulante particulièrement intéressantes.

Les héparosanes-N,O-sulfatés de la présente invention sont très peu toxiques ; leur toxicité est parfaitement compatible avec leur utilisation comme médicaments.

L'invention s'étend donc aussi aux compositions pharmaceutiques renfermant comme principe actif un héparosane-N,O-sulfaté, objet de la présente invention, un de ses sels, ou une composition d'héparosane N,O-sulfaté contenant au moins 70 % de cet héparosane-N,O-sulfaté ou l'un de ses sels, en association avec un ou plusieurs véhicules pharmaceutiques appropriés.

Ces compositions pharmaceutiques sont utiles notamment pour le traitement, à titre préventif ou curatif, des troubles de la paroi vasculaire tels que l'athérosclérose et l'artériosclérose et des états d'hypercoagulabilité observés par exemple à la suite d'opérations chirurgicales, de développements tumoraux ou de dérèglements de la coagulation induits par des activateurs enzymatiques, bactériens ou viraux.

La posologie peut largement varier en fonction de l'âge, du poids et de l'état de santé du patient, de la nature et de la sévérité de l'affection ainsi que de la voie d'administration. Cette posologie comprend l'administration soit d'une ou plusieurs doses d'environ 1 mg à 1 g par jour, de préférence d'environ 5 mg à 500 mg par jour par exemple de l'ordre de 200 mg par jour par voie intraveineuse, ou par voie sous-cutanée, en administrations discontinues ou à intervalles réguliers, soit d'une dose journalière de l'ordre de 200 mg à 1000 mg par jour par voie orale.

Ces doses peuvent être naturellement ajustées pour chaque patient en fonction des résultats observés et des analyses de sang effectuées auparavant.

Les N-acétylhéparosanes (composés de formule II), les héparosanes (composés de formule III) et les héparosanes N-sulfatés (composés de formule IV) issus respectivement des étapes b, c et d dans la préparation des produits de départ du procédé de la présente invention, les héparosanes-N,O-sulfatés objet de la présente invention, ainsi que les fractions de tous ces produits, constituent des intermédiaires de haute masse moléculaire utiles pour la préparation par dépolymérisation des N-acétylhéparosanes, des héparosanes, des héparosanes-N-sulfatés et des héparosanes-N,O-sulfatés de faible masse moléculaire, inférieure à $1,5.10^4$ Da et notamment comprise entre $1,5.10^3$ et $1,5.10^4$ Da.

La dépolymérisation peut-être effectuée selon les méthodes décrites dans la littérature pour préparer les héparines de bas poids moléculaire, par exemple, par dépolymérisation au periodate et notamment selon la méthode décrite dans la demande de brevet EP-0 287 477, par dépolymérisation par des radicaux libres et notamment selon la méthode décrite dans le brevet EP-0 121 067. La dépolymérisation peut aussi être réalisée par β-élimination et plus particulièrement selon la méthode décrite dans la demande de brevet EP-0 040 144. Dans ce cas, on obtient à l'extrémité non réductrice, une terminaison acide uronique α,β-insaturé de formule (a), éventuellement O-sulfaté.

Comme méthode de dépolymérisation, est aussi utilisée de manière préférentielle, la dépolymérisation par action de l'acide nitreux, et notamment celle décrite dans le brevet EP-0 037 319. Cette méthode conduit dans un premier temps à des produits ayant à l'extrémité réductrice, une unité 2,5-anhydromannose de formule (b) :

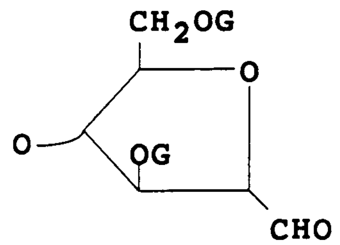

(b)

dans laquelle, G représente un atome d'hydrogène ou un atome d'hydrogène et un groupe sulfate, et ceci en fonction des produits mis en oeuvre lors de la dépolymérisation (G est égal à un atome d'hydrogène pour les composés de formules III ou IV et G est égal à un atome d'hydrogène et un groupe sulfate pour les produits de formule I), puis, lorsque la dépolymérisation est suivie d'une réduction, on obtient des fragments ayant à l'extrémité réductrice, une unité 2,5-anhydromannitol de formule (c) :

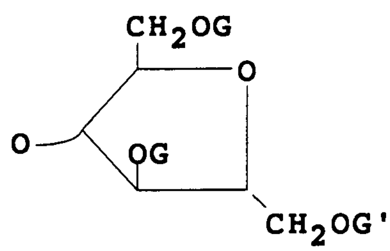

(c)

dans laquelle G' représente un atome d'hydrogène lorsque la dépolymérisation n'est pas suivie d'une sulfatation, ou un groupe sulfate lorsque la dépolymérisation est suivie d'une sulfatation.

Lorsque la dépolymérisation par action de l'acide nitreux est suivie d'une oxydation, on obtient des fragments ayant à l'extrémité réductrice une unité acide 2,5-anhydromannonique de formule (d) :

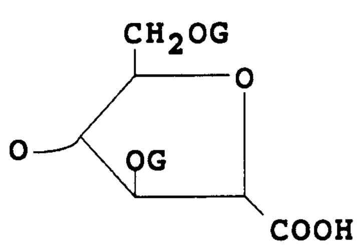

(d)

La dépolymérisation peut aussi éventuellement être effectuée par des méthodes enzymatiques comme par exemple celles indiquées dans les demandes de brevets EP-0 244 235 et EP-0 244 236.

Les méthodes de dépolymérisation indiquées ci-dessus sont données à titre d'exemples et ne sont pas limitatives. On peut utiliser toute autre méthode de dépolymérisation de glycosaminoglycanes. Par la suite, les produits de dépolymérisation peuvent être soumis à un ou plusieurs fractionnements afin d'obtenir des N-acétylhéparosanes, des héparosanes, des héparosanes-N-sulfatés et des héparosanes-N,O-sulfatés constitués de chaînes de faible masse moléculaire.

La présente invention a aussi pour objet l'utilisation des N-acétylhéparosanes de haute masse moléculaire (composés de formule II), les héparosanes (composés de formule III), les héparosanes N-sulfatés (composés de formule IV) ainsi que les héparosanes-N,O-sulfatés (composés de formule I) de haute masse moléculaire, objet de la présente invention, pour l'obtention après dépolymérisation de N-acétylhéparosanes, d'héparosanes, d'héparosanes N-sulfatés, d'héparosanes-N,O-sulfatés de faible masse moléculaire, inférieure à 1,5 $10^4$ Da.

La présente invention a aussi plus particulièrement pour objet, l'utilisation d'héparosanes N-sulfatés (composés de formule IV), d'héparosanes-N,O-sulfatés (composés de formule I) pour la préparation, après fragmentation, des héparosanes-N,O-sulfatés de faible masse moléculaire, inférieure à 1,5 $10^4$ Da, et notamment comprise entre 1,5. $10^3$ Da et 1,5. $10^4$ Da.

Lorsque l'on utilise, lors de la fragmentation comme produits de départ, les composés de formule IV, les héparosanes-N,O-sulfatés de faible masse moléculaire sont obtenus en soumettant les héparosanes N-sulfatés de faible masse moléculaire obtenus par la fragmentation des produits de formule IV soit à une N,O-sulfatation partielle, soit à une N,O-sulfatation partielle suivie d'une étape de N-sulfatation totale.

L'invention est illustrée par les exemples ci-après.

## PREPARATIONS

### PREPARATION I

#### Préparation d'un N-acétylhéparosane de haute masse moléculaire (Procédé I)

**1) Culture de la souche bactérienne Escherichia coli (K5) et séparation d'un filtrat contenant du N-acétylhéparosane**

On ensemence 400 ml du milieu B, de composition précisée dans le tableau I ci-après, avec la souche Escherichia coli SEBR 3282 (déposée auprès de la CNCM de l'Institut Pasteur, Paris France, sous le N° I-1013) et on incube sous agitation pendant 2 h à 37°C.

La préculture obtenue est alors transférée dans un fermenteur de 18,5 l contenant 11 l du milieu A, de composition précisée dans le tableau I ci-après et on incube pendant 6 h 30 min. à 37°C et à pH égal à 7,2, la pression partielle en oxygène étant maintenue à 40 mmHg par régulation de l'injection d'air (jusqu'à 20 l/min.) et de l'agitation. On ajoute alors du glycérol en introduisant de façon continue une solution stérile contenant 500 g/l de glycérol à raison de 18 g/h pendant 16-17 h.

On poursuit la culture dans les mêmes conditions de température, de pH et de pression partielle en oxygène jusqu'à consommation quasi totale du glycérol. Le suivi de la DO ($\lambda$ = 600 nm) du milieu de la suspension de culture après la fin de l'ajout en glycérol, montre un état stationnaire ou de lyse légère jusqu'à l'arrêt de la culture à 28-30 h d'âge en fermenteur.

On refroidit alors le bouillon de culture à 25°C puis on le filtre à travers une membrane de porosité 0,22 μm. On obtient ainsi environ 12 l de filtrat contenant du N-acétylhéparosane.

## TABLEAU I

Composition et préparation du milieu A et du milieu B.

### MILIEU A

Dans 900 ml d'eau ultra-purifiée dissoudre dans l'ordre :

| | | |
|---|---|---|
| NTA (acide nitrilotriacétique) | 1000 | mg |
| $K_2HPO_4$ | 790 | mg |
| Acide glutamique | 11000 | mg |
| $MgCl_2$, $6H_2O$ | 500 | mg |
| $K_2SO_4$ | 450 | mg |
| $FeSO_4$, $7H_2O$ | 18 | mg |
| $CaCl_2$, $2H_2O$ | 2 | mg |
| NaCl | 500 | mg |
| KCl | 5000 | mg |
| Solution d'oligo-éléments (cf. Tableau II) | 1 | ml |
| Glycérol | 10000 | mg |

Ajuster le pH à 7,2 avec de la potasse concentrée de densité 1,38 et compléter à 1000 ml avec de l'eau ultra-purifiée. Effectuer une filtration stérilisante sur membrane de 0,2 µm.

Solution de glycérol

Dissoudre 50 g de glycérol dans une quantité adéquate d'eau ultra-purifiée et ajuster le volume à 1000 ml avec le même solvant. Effectuer une filtration stérilisante sur membrane de 0,2 µm.

L'anti-mousse employé en cours de fermentation est le Struktol J 673 (Schill et Seilacher®).

### MILIEU B

La préparation du milieu B est identique à celle du milieu A, à la différence près, qu'il convient d'ajouter en plus le tampon (pH 7,2) : acide 3-morpholinopropanesulfonique (M.O.P.S.) après addition de l'agent anti-mousse.

## TABLEAU II

### Préparation de la solution d'oligo-éléments utilisée dans la préparation du milieu A et du milieu B

dans 800 ml d'eau ultra-purifiée dissoudre (dans l'ordre) :

| | |
|---|---|
| $H_3BO_3$ | 500mg |
| $Na_2MoO_4,2H_2O$ | 1930mg |
| $CoCl_2,6H_2O$ | 11850mg |
| $CuSO_4,5H_2O$ | 25mg |
| $ZnSO_4,7H_2O$ | 2000mg |
| $AlCl_3,6H_2O$ | 2410mg |

Ajouter 100 ml d'acide chlorhydrique de densité 1,19 et compléter à 1000 ml avec de l'eau ultra-purifiée.

**2) Isolement et purification d'un N-acétylhéparosane majoritairement de haute masse moléculaire :**

Etape a - Précipitation éthanolique :

On ajoute au filtrat environ 48 l d'éthanol à 95 % (v/v) et on laisse le mélange précipiter et décanter à 4°C pendant 8 h.

On élimine le sumageant par pompage, puis centrifugation, et on reprend le culot de centrifugation dans environ 1 l d'eau ultra-purifiée.

Etape b - Dialyse :

On dialyse pendant 24 h la solution obtenue à l'étape précédente placée dans un boudin NOJAX 40 composé d'une membrane à base de cellulose et de porosité 24 Å, contre de l'eau ultra-purifiée (1 volume de solution / 6 volumes d'eau, renouvelés après 2 h, 8 h et 16 h).

Cette opération permet d'éliminer les petites molécules présentes dans le milieu de culture, telles que sels, sucres, acides aminés, oligonucléotides et oligopeptides.

Etape c - Précipitation, déshydratation et séchage :

On ajoute à 1 volume de la solution dialysée, 0,5 M de NaCl et 4 volumes d'éthanol. On laisse se former le précipité pendant 5 min. à température ambiante. On centrifuge à 5000 g pendant 20 min.

On reprend les culots de centrifugation dans l'éthanol, on agite la suspension obtenue, et on la laisse reposer pendant 1 h à température ambiante. On répète les opérations de centrifugation et de mise en suspension. On centrifuge à nouveau à 5000 g pendant 20 min. On sèche les culots de centrifugation obtenus dans une étuve sous vide à 40°C pendant 24 h.

Etape d - Broyage en poudre :

Les culots de centrifugation secs sont broyés à l'aide d'un mortier dans des conditions anhydres.

Etape e - Chromatographie d'échange d'anions :

On reprend les culots de centrifugation broyés, dans un tampon appelé tampon D, de composition Tris-HCl 20 mM pH 7,5, à raison de 100 ml/g.

La solution obtenue est chromatographiée sur une colonne d'échange d'anions forts comportant une matrice d'agarose réticulée avec des groupes ammonium quaternaires (le "Q-Sepharose fast flow" de Pharmacia®) préalablement équilibrée avec le tampon D, à raison de 50 ml de gel pour 1 g de poudre.

On lave le gel avec une quantité suffisante de tampon D pour le retour à la ligne de base de la détection UV à 214 nm, puis avec une solution de pipérazine 25 mM dont le pH a été ajusté à 3,5.

On élue avec une solution de pH 3,5 ayant une composition : NaCl 0,5 M et pipérazine 25 mM. L'éluat est neutralisé à l'aide d'une solution de NaOH 5 N.

Etape f - Précipitation, déshydratation, séchage et broyage :

On répète les opérations décrites dans les étapes c et d ci-dessus, sans ajout de chlorure de sodium.

Le N-acétylhéparosane obtenu à l'issue de l'étape f, est appelé lot A.

**3) Caractérisation du N-acétylhéparosane obtenu à l'issue des différentes étapes de purification :**

Spectre de résonance magnétique nucléaire (RMN)

Les spectres RMN du proton et du carbone $^{13}C$ sont comparés à ceux du N-acétylhéparosane décrit par W. F. Vann (Eur. J. Biochem., (1981), 116, 359-364).

L'étude des spectres obtenus avec le N-acétylhéparosane du lot A confirme l'identité chimique du produit au N-acétylhéparosane décrit par W.F. Vann. Il s'agit de chaînes de polymères constituées de structures répétées de β-D-glucuronyl-1,4-α-N-acétyl-D-glucosaminyl-(1,4).

Détermination de la répartition des masses moléculaires par chromatographie d'exclusion stérique

Première méthode

La répartition des masses moléculaires est déterminée par HPLC d'exclusion dans les conditions suivantes :

* Colonne constituée de billes de silice de diamètre 10 μm et de porosité 250 Å.
* Eluant: solution aqueuse de sulfate de sodium 0,5 M.
* Débit : 1 ml/min.
* Détection UV à $\lambda$ = 205 nm.
* L'étalonnage est effectué à l'aide d'une gamme d'oligosaccharides dérivés de l'héparine, de masses moléculaires suivantes : 1324, 1883, 2436, 3411, 3996, 4535, 4995, 5365, 6150, 6671, 7542, 8655, 10088, 11561, 12950, 14809, 17387 et 22674 Da.

Compte tenu de cette gamme d'étalons, seules les masses moléculaires entre 930 Da et 57000 Da sont évaluées de manière précise. On admet que la densité optique détectée est proportionnelle à la quantité de N-acétylhéparosane. La précision de la méthode diminue de manière importante pour les hautes masses moléculaires et notamment supérieures à $5.10^4$ Da.

Le profil d'élution de la chromatographie d'exclusion du lot A permet de constater que la distribution est polydispersée et qu'elle comporte un pic majoritaire à environ $2.10^5$ Da. Une fraction pondérale au moins égale à 70 % du lot A possède une masse comprise entre $1,0.10^5$ et $3,0.10^5$ Da.

Deuxième méthode

Les résultats ont été vérifiés par HPLC d'exclusion en utilisant un détecteur par diffusion de lumière.

* Colonnes : Système composé d'une pré-colonne et de trois colonnes successives :

  - PL-Aquagel-OH® (pré-colonne)
  - PL-Aquagel-OH 40®
  - PL-Aquagel-OH 50®

- PL-Aquagel-OH 60®

* Eluant : Solution aqueuse de $NaN_3$ 400 ppm
* Débit : 1 ml/min.
* Température ambiante
* Détection : Couplage réfractomètre différentiel/diffusion de la lumière

La réponse du détecteur de masse (diffusion de la lumière) est proportionnelle à :

$$C \times (dn/dc)^2 \times Mw$$

C = concentration
dn/dc = incrément de l'indice de réfraction
Mw = masse moléculaire moyenne

Le calcul du rapport dn sur dc a été effectué à partir des réponses du réfractomètre différentiel en tenant compte de celles de deux échantillons de référence (Dextran Mw = 70000 Da et 150000 Da).
Cette méthode a confirmé qu'une fraction pondérale au moins égale à 70 % du lot A possède une masse comprise entre $1,0.10^5$ et $3,0.10^5$ Da et que le profil d'élution obtenu après chromatographie d'exclusion du lot A, comporte un pic majoritaire à environ 210000 Da.

Suivi de la répartition des masses moléculaires par électrophorèse sur gel de polyacrylamide

On a soumis ces échantillons à analyser ainsi qu'un marqueur de fin de migration comprenant du bleu de bromophénol, à une électrophorèse en tampon Tris-borate dans un gel de polyacrylamide à 15 % obtenu par polymérisation d'un mélange 29/1 d'acrylamide et de N,N'-méthylène-bis-acrylamide.
La migration est effectuée sous 40 mA pendant environ 4 h sur un gel de 18 cm de longueur jusqu'à la sortie du marqueur de fin de migration. Le gel est alors coloré au bleu alcian, puis à l'argent, selon la technique de S. Pelkonen et al (J. Bact., (1983), 170, 6, 2646) spécifique aux polysaccharides acides.
Cette analyse par électrophorèse a été effectuée sur le produit partiellement purifié obtenu à l'issue de l'étape a et sur le produit purifié, issu de l'étape finale, dans le but de vérifier l'absence de modification importante de la répartition des masses moléculaires du N-acétylhéparosane au cours de la purification.
L'observation des profils obtenus pour le produit partiellement purifié obtenu à l'issue de l'étape a et pour le produit purifié à l'étape finale, ne met pas en évidence de différences importantes (présence de bandes d'intensité comparable aux mêmes distances de migration). Il n'y a donc pas de modification importante de la répartition des masses moléculaires du N-acétylhéparosane au cours de sa purification.

Dosage des acides uroniques

La quantité d'acide uronique par unité de masse du produit purifié, obtenu à l'issue de l'étape finale, à été déterminée par colorimétrie selon la méthode décrite par T. Bitter, (Analytical Biochemistry, (1962), 4, 330-334). Cette méthode de dosage est basée sur la réaction des glycosaminoglycanes avec le carbazole en milieu acide à chaud, qui provoque une coloration en rose proportionnelle à la quantité d'acide uronique libérée. Dans ce cas précis, il s'agit de l'acide glucuronique.
Pour le lot A, le produit partiellement purifié obtenu à l'issue de l'étape finale à un taux d'acide uronique de 2,2 µmole/mg.

Spectrophotométrie dans le domaine ultraviolet et visible

Le produit purifié (lot A), est dissous dans de l'eau ultra-purifiée et la solution obtenue (C = 1 mg/ml) est placée dans une cuve de 1 cm de trajet optique. Son spectre d'absorption est enregistré entre 200 et 600 nm.
Le spectre obtenu permet d'affirmer, en particulier sur la base de l'absorption à 256 nm, que le lot A contient moins de 1 % d'ADN.

Dosage des protéines totales

Le kit "protein assay" commercialisé par BIORAD® est utilisé pour doser les protéines totales. La méthode de dosage est basée sur le fait que la longueur d'onde du maximum d'absorbance d'une solution acide de bleu brillant de Coomassie g-250, passe de 465 nm à 595 nm, lorsque des protéines viennent s'y fixer (Reisner et al., Anal Biochem, (1975), 64, 509).

Le taux de protéines totales du lot A est inférieur à 1,5 %.

Dosage des groupes amino libres ($NH_2$)

Ce dosage a été effectué selon la méthode décrite par Zensaku Yosizawa et al., dans Biochemica et Biophysica Acta, (1967), 141, 358-365.

Le paramètre taux de $NH_2$ (exprimé en µmole/mg) est un indicateur de quantités d'unités β-D-glucuronyl-1,4-α-N-acétyl-D-glucosaminyl-(1,4) désacétylées et d'éventuel les impuretés présentes, qui contiennent un groupement amino libre.

Le lot A à un taux de $NH_2$ de 0,1 µmole/mg.

**PREPARATION II**

**Préparation d'un N-acéthylhéparosane majoritairement de haute masse moléculaire (Procédé II)**

**1) Culture de la souche bactérienne Escherichia coli (K5) et séparation d'un filtrat contenant du N-acétylhéparosane**

La culture de la souche Escherichia coli SEBR 3282 et la séparation d'un filtrat contenant du N-acétylhéparosane ont été réalisées selon la méthode décrite dans la Préparation I.

**2) Isolement et purification d'un N-acétylhéparosane majoritairement de haute masse moléculaire**

Etape a - Dialyse

375 ml de filtrat sont soumis à une dialyse selon le procédé décrit dans la Préparation I, [2) Isolement et purification d'un N-acétylhéparosane majoritairement de haute masse moléculaire, Etape b]. Après dialyse on obtient environ 1020 ml de solution purifiée.

Etape b - Purification en milieu acide

On ajoute à la solution dialysée une quantité adéquate d'une solution d'HCl 5 N pour obtenir un pH égal à 3,5. On élimine par centrifugation le précipité formé, puis on acidifie la solution avec le même acide (HCl 5N) pour obtenir un pH égal à 1,8. Un précipité peut se former, qui sera éliminé par centrifugation.

On neutralise ensuite la solution à l'aide d'une solution de NaOH 5 N.

Etape c - Précipitation, déshydratation et séchage

On ajoute à la solution neutralisée une quantité adéquate de chlorure de sodium pour avoir une solution de 0,5 M en NaCl, puis on ajoute 4 volumes d'éthanol. On laisse se former le précipité pendant 5 min. à température ambiante. On centrifuge à 5000 g pendant 20 min.

On reprend les culots de centrifugation dans l'éthanol, on agite la suspension obtenue, et on la laisse reposer pendant 1 h à température ambiante. On répète les opérations de centrifugation et de mise en suspension. On centrifuge à nouveau à 5000 g pendant 20 min.

On sèche les culots de centrifugation obtenus dans une étuve sous vide à 40°C pendant 24 h.

Etape d - Hydrolyse alcaline et dialyse

Le produit obtenu à l'étape précédente après séchage est mis en solution à 2,5 % (p/v) dans une solution de NaOH 0,25 N. On maintient la solution ainsi obtenue 2 heures à 50 °C.

On neutralise ensuite à l'aide d'une solution d'HCl 5 N et on soumet ensuite la solution contenant le polysaccharide à une dialyse selon le procédé décrit dans la Préparation I, [ 2) Isolement et purification d'un N-acétylhéparosane majoritairement de haute masse moléculaire, Etape b].

Après dialyse, on obtient environ 990 ml de solution.

Etape e - Chromatographie d'échange d'anions

A la solution dialysée on ajoute des quantités adéquates de pipérazine, d'EDTA et de Triton X-100 (Prolabo®) pour avoir respectivement des concentrations de 25 mM en pipérazine, 2 mM en EDTA et 0,2 % (v/v) en triton X-100.

Le pH est ensuite ajusté à 3,5 à l'aide d'une solution d'HCl 5 N. On dépose cette solution sur une colonne Q-Sepharose Fast Flow de 400 ml, équilibrée dans un tampon pipérazine contenant 25 mM de pipérazine, 2 mM d'EDTA et 0,2 mM de triton X-100 (pH = 3,5).

On lave par le tampon pipérazine, on élue avec une solution de NaCl 0,5 M, puis on précipite par 4 volumes d'éthanol.

On sèche sous vide à 40°C. On obtient ainsi environ 9,85 g de N-acétylhéparosane.

Etape f - Chromatographie d'exclusion stérique

4 g du produit obtenu au stade précédent sont mis en solution dans 60 ml d'une solution tampon de composition tris-HCl 20 mM pH 7,5 et NaCl 1 M, puis passés sur une colonne de 200 ml d'octyl Sepharose® préalablement équilibrée avec le même tampon.

A la fraction non retenue, on ajoute 4 volumes d'éthanol. On lave le précipité formé et on le sèche à 40°C sous vide.

On obtient ainsi 3,90 g de N-acétylhéparosane (lot B).

**3) Caractérisation du N-acétylhéparosane obtenu à l'issue des différentes étapes de purification**

Spectre de résonance magnétique nucléaire RMN

L'étude des spectres RMN du proton et du carbone $^{13}C$ obtenus avec cet N-acétylhéparosane, confirme l'identité chimique du produit au N-acétylhéparosane décrit par W.F. Vann (Eur. J. Biochem. (1981) 116, 359-364).

Détermination de la répartition des masses moléculaires par chromatographie d'exclusion stérique

La répartition des masses moléculaires est déterminée par HPLC d'exclusion stérique selon les méthodes utilisées pour la détermination de la répartition des masses moléculaires de N-acétylhéparosanes décrites dans la Préparation I. Une fraction pondérale au moins égale à 60 % des chaînes qui constituent le lot B issu de la Préparation II possède une masse moléculaire comprise entre $5,0.10^4$ et $2,5.10^5$ Da.

Dosage des acides uroniques

Le N-acétylhéparosane issu de l'étape e, a un taux d'acide uronique de 2,2 µmole/mg.

Spectrophotométrie dans le domaine de l'ultraviolet et visible

Le spectre obtenu permet d'affirmer que le N-acétylhéparosane obtenu contient moins de 1 % d'ADN.

Dosage des protéines totales

Le taux de protéines totales du lot B est inférieur à 1 %.

Dosage des groupes amino libres ($NH_2$)

Le taux de $NH_2$ est inférieur à 0, 1 µmole/mg

**PREPARATION III**

**Préparation d'un N-acétylhéparosane majoritairement de haute masse moléculaire (Procédé III)**

**1) Culture de la souche bactérienne Escherichia coli (K5)**

La culture de la souche Escherichia coli SEBR 3282 a été réalisée selon la méthode décrite dans la Préparation I. On obtient environ 12 l de culture contenant du N-acétylhéparosane.

**2) Purification préliminaire**

Etape a - Centrifugation

A la fin de la culture, on centrifuge la suspension obtenue à 8000 t/min. (soit entre 11000 et 14000 g) pendant 20 min.

Etape b - Mise en contact avec une solution alcaline

Après la centrifugation, le culot est éliminé et le surnageant est mis en contact avec une solution de NaOH 0,1 N pendant environ 1 heure.

Etape c - Préfiltration

La solution obtenue à l'étape précédente est soumise à une préfiltration sur filtre 3M® série 300 en polypropylène.

Etape d - Concentration sur membrane de seuil de coupure déterminé.

Le filtrat obtenu à l'étape c est concentré sur cartouche à fibres creuses Amicon®, seuil de coupure 30 000 Da ou équivalent. On obtient ainsi une solution concentrée en N-acétylhéparosane de haute masse moléculaire.

Etape e - Dialyse

On dialyse la solution concentrée en N-acétylhéparosane de haute masse moléculaire contre de l'eau ultra-purifiée toujours sur système Amicon®, à un facteur de dilution très élevé (> 10000).

**3) Isolement et purification d'un N-acétylhéparosane majoritairement de haute masse moléculaire :**

On procède comme il est indiqué dans la Préparation I [ 2) Isolement et purification d'un N-acétylhéparosane majoritairement de haute masse moléculaire, Etape c - Etape f] et on obtient un N-acétylhéparosane (lot C) ayant des caractéristiques similaires à celles du lot A, ou comme il est indiqué dans la Préparation II [ 2) Isolement et purification d'un N-acétylhéparosane majoritairement de haute masse moléculaire, Etape a - Etape f].

**PREPARATION IV**

**Préparation d'un N-acétylhéparosane majoritairement de haute masse moléculaire (Procédé IV)**

**1) Culture de la souche bactérienne Escherichia coli (K5)**

La culture de la souche Escherichia coli SEBR 3282 a été réalisée selon la méthode décrite dans la Préparation I en utilisant comme milieu de culture le milieu C au lieu du milieu A, et le milieu D au lieu du milieu 19.
La composition du milieu C et du milieu D est précisée dans le tableau III (cf. page suivante).
On obtient ainsi environ 12 l de filtrat contenant du N-acétylhéparosane.

**2) Isolement et purification d'un N-acétylhéparosane majoritairement de haute masse moléculaire**

On procède comme il est décrit dans la Préparation I, [2) Isolement et purification d'un N-acétylhéparosane majoritairement de haute masse moléculaire, Etape a, Etape f]. On obtient ainsi le lot D.

**3) Caractérisation du N-acétylhéparosane obtenu**

Détermination de la répartition des masses moléculaires par chromatographie d'exclusion

Les méthodes utilisées sont identiques à celles décrites dans la Préparation I [ 3) Caractérisation du N-acétylhéparosane obtenu à l'issue des différentes étapes de purification : Détermination de la répartition des masses moléculaires par chromatographie d'exclusion stérique - Première et deuxième méthode].

L'examen du profil d'élution de la chromatographie d'exclusion stérique du lot D, permet de constater que la distribution des masses moléculaires est polydispersée et qu'elle comporte un pic majoritaire à environ 110 000 Da.

Une fraction pondérale au moins égale à 75 % du lot D possède une masse comprise entre $1,0.10^4$ et $2,5.10^5$ Da.

Dosage des acides uroniques

Le taux d'acide uronique de 2,0 µmole/mg.

Dosage des groupes amino libres ($NH_2$)

Le taux de $NH_2$ du lot est inférieur à 0,1 µmole/mg

## TABLEAU III

### Composition et préparation du milieu C et du milieu D

**MILIEU C**

Le milieu C est préparé par la réunion des trois solutions stériles ci-dessous :

Solution N°1

dans 700 ml d'eau ultra-purifiée dissoudre dans l'ordre :

| | |
|---|---|
| Complexant : N'[Tris-(hydroxyméthyl) méthyl] glycine (Tricine commercialisé par Fluka®) | 360 mg |
| $FeSO_4$, $7H_2O$ | 280 mg |
| $CaCl_2$, $2H_2O$ | 6,7 mg |
| $MgCl_2$, $6H_2O$ | 1270 mg |
| $K_2SO_4$ | 500 mg |
| KCl | 5000 mg |
| Hydrolysat de caséine (source principale d'acides aminés) HY CASE SF (commercialisé par Sheffield®) | 25000 mg |
| Extrait de levure (commercialisé par Difco®) | 18000 mg |
| Solution d'oligo-éléments (cf tableau II, Préparation I) | 1 ml |

Agent antimousse Struktol J673 (commercialisé par Schill et Seilacher®) : quelques gouttes à la pipette Pasteur.

Ajuster le pH à 7,4 avec une solution de KOH (d = 1,38) et compléter à 850 ml avec de l'eau ultra-purifiée. Autoclaver le milieu 45 min à 120°C.

Solution N°2

Dans environ 40 ml d'eau ultra-purifiée, dissoudre 5 g de $K_2HPO_4$ puis ajuster à 50 ml avec le même solvant. Filtrer la solution obtenue à travers un filtre de porosité 0,2 $\mu$m.

Solution N°3

Dissoudre 20,7 g de glycérol dans une quantité adéquate d'eau ultra-purifiée et ajuster le volume à 100 ml avec le même solvant. Autoclaver à 110°C pendant 30 minutes.

**MILIEU D**

La préparation du milieu D est identique à celle du milieu C, à la différence près, qu'il convient d'ajouter en plus 20 g de tampon (pH = 7,2) : acide 3-morpholinopropanesulfonique après l'addition de l'agent anti-mousse.

**PREPARATION V**

**Préparation d'un héparosane (dérivé N-désacétylé à 40 %)**

On dissout 3,7 g du N-acétylhéparosane décrit dans la préparation IV (lot D) dans 74 ml de NaOH 1 N. On porte la solution à 50°C et on laisse réagir à cette température pendant 8 heures sous agitation et sous azote. On ajuste ensuite le pH à environ 8 à l'aide d'une solution d'HCl 2N, on dialyse la solution obtenue contre de l'eau ultra-purifiée, puis on la lyophilise.

Après la lyophilisation, on obtient 2,91 g de produit (lot E).

Taux de N-désacétylation

Le dosage des groupes amino libres ($NH_2$) du produit obtenu après lyophilisation indique que le N-acétylhéparosane à été N-désacétylé à 40 %.

**PREPARATION VI**

**Préparation d'un héparosane N-sulfaté (dérivé N-désacétylé à 80 %)**

Comme matière première pour les différentes modifications chimiques, on utilise un N-acétylhéparosane appelé lot F préparé selon le procédé décrit dans la Préparation II.

Le taux d'acide uronique de ce produit est de 2,41 μmole/mg.

Etape a- N-Désacétylation partielle

On dissout 1,9 g du lot F dans 38,5 ml de NaOH 2 N. On porte la solution à 50°C et on laisse réagir à cette température pendant 8 heures sous azote.

On ajuste ensuite le pH à 8,25 par addition de HCl 2 N. On dialyse contre de l'eau ultra-purifiée puis on lyophilise.

On obtient ainsi 1,6 g de produit.

Taux de N-désacétylation

Le dosage des groupes amino-libres ($NH_2$) indique que le N-acétylhéparosane a été N-désacétylé à 80 %.

Etape b - N-sulfatation

On dissout 1,33 g du produit obtenu à l'étape précédente dans 57 ml d'eau ultra-purifiée, on ajoute 1,9 g de $Na_2CO_3$ et 1,9 g de complexe trioxyde de soufre-triméthylamine et on porte à 55°C pendant 20 heures. On ajuste ensuite la conductivité de la solution à celle d'une solution de NaCl 0,5 M par addition d'eau déminéralisée et on précipite par 4 volumes d'éthanol. On centrifuge, on reprend le culot avec une solution de NaCl 0,5 M, on précipite par 4 volumes d'éthanol et on centrifuge.

On dissout le culot dans l'eau ultra-purifiée et on dialyse contre de l'eau ultra-purifiée selon le procédé décrit dans la Préparation I puis, on lyophilise.

On obtient ainsi 1,809 g d'héparosane N-sulfaté (lot F1).

**PREPARATION VII**

**Préparation d'un héparosane N-sulfaté (dérivé N-désacétylé à 80 %) de faible masse moléculaire par dépolymérisation par l'acide nitreux ; (Concentration finale en nitrite : 0,04 M)**

Etape a- Fragmentation par le nitrite de sodium

1 g du produit obtenu à la Préparation VI est dissous dans 8 ml d'eau ultra purifiée. Le pH de la solution est ajusté à 2,5 à l'aide d'HCl 2 N. A la solution agitée vigoureusement à température ambiante sous flux d'azote, on ajoute 0,276 ml d'une solution aqueuse de nitrite de sodium à 100 mg/ml (molarité finale en nitrite : 0,04 M). Le pH est immédiatement réajusté à 2,5 à l'aide d'HCl 2 N et le volume total de la solution est complété à 10 ml. L'agitation est maintenue 2 heures sous flux d'azote.

Etape b- Réduction des groupements 2,5-anhydromannose formés

On ajuste le pH de la solution à 7,0 à l'aide de soude 2 N, puis on ajoute 0,2 g de borohydrure de sodium et l'on agite 15 heures à l'air libre, à température ambiante.
Le borohydrure en excès est hydrolysé par acidification à pH 3,5 à l'aide d'acide acétique à 10 %. On soumet la solution réactionnelle à une agitation pendant 15 minutes, puis on ajuste le pH à 7,0 à l'aide de soude 2 N.
Le produit de la réaction est précipité par 4 volumes d'éthanol, centrifugé, lavé à l'éthanol pur et séché sous vide à 60 °C.
On obtient ainsi 980 mg d'héparosane N-sulfaté (dérivés N-désacétylé à 80%) dépolymérisé, de faible masse moléculaire ayant à l'extrémité réductrice, un groupement 2,5 anhydromannitol (lot G).

Détermination de la répartition des masses moléculaires par HPLC d'exclusion stérique

La répartition des masses moléculaires est déterminée par HPLC d'exclusion stérique dans les conditions suivantes :

Colonne TSK 2000 (TOYO SODA).
Phase mobile : Solution aqueuse de sulfate de sodium 0,5 M
Débit : 1 ml/min.
Détecteur UV à $\lambda$ = 205 nm
L'étalonnage est effectué à l'aide d'une gamme d'oligosaccharides, dérivés de l'héparine comme décrit dans la Préparation I [3- Caractérisation du N-acétylhéparosane obtenu à l'issue des différents étapes de purification : Détermination de la répartition des masses moléculaires par chromatographie d'exclusion stérique - Première méthode].

Certains résultats déduits du profil d'élution sont rassemblés dans le tableau IV ci-après.

$PM_1$ représente la masse moléculaire telle qu'une fraction pondérale de 1 % du produit ait une masse moléculaire supérieure à $PM_1$.
$PM_2$ représente la masse moléculaire telle qu'une fraction pondérale de 10 % du produit ait une masse moléculaire supérieure à $PM_2$.
$PM_4$ représente la masse moléculaire telle qu'une fraction pondérale de 10 % du produit ait une masse moléculaire inférieure à $PM_4$.
$PM_5$ représente la masse moléculaire telle qu'une fraction pondérale de 1 % du produit ait une masse moléculaire inférieure à $PM_5$.
$PM_3$ représente la masse moléculaire correspondant au maximum d'absorption.

## TABLEAU IV

### Répartition des masses moléculaires du produit obtenu à la Préparation VII (Lot G)

| $PM_1$ | $PM_2$ | $PM_3$ | $PM_4$ | $PM_5$ |
|---|---|---|---|---|
| 16939 | 8843 | 3716 | 1757 | 925 |

**PREPARATION VIII**

**Préparation d'un héparosane N-sulfaté (dérivé N-désacétylé à 80 %) de faible masse moléculaire par dépolymérisation par l'acide nitreux ; (Concentration finale en nitrite : 0,03 M)**

On procède comme il est décrit dans la Préparation VII, en ajoutant 0,208 ml d'une solution de nitrite de sodium à 100 mg/ml au lieu de 0,276 ml.
On obtient ainsi 990 mg de produit (lot H).
La répartition des masses moléculaires est déterminée par HPLC d'exclusion stérique, comme décrit dans la Préparation VII. Certains résultats déduits des profils d'élution sont rassemblés dans le Tableau V. $PM_1$, $PM_2$, $PM_3$, $PM_4$ et $PM_5$, ont les significations données pour le Tableau IV.

**TABLEAU V**

Répartition des masses moléculaires du produit obtenu à la Préparation VIII (Lot H)

| $PM_1$ | $PM_2$ | $PM_3$ | $PM_4$ | $PM_5$ |
|---|---|---|---|---|
| 19856 | 11146 | 5377 | 2460 | 1536 |

**HEPAROSANES-N,O-SULFATES**

**EXEMPLE 1**

**Héparosanes-N,O-sulfatés ; dérivés N-désacétylés à 80 % ayant un taux de sulfatation de 2,6**

Etape a - Formation du sel de tetrabutylammonium

On dissout 800 mg de l'héparosane N-sulfaté obtenu selon la Préparation VI (le N-acétylhéparosane utilisé comme matière première étant préparé selon la Préparation IV), dans 100 ml d'eau. On dépose cette solution sur une colonne d'échange d'ions à base de polystyrène réticulé par le divinylbenzène (Dowex 50 W 8 de Dow Chemical® 20-50 Mesh) préalablement conditionnée en milieu acide, de façon à régénérer la forme acide du produit. Le pH de l'éluat est amené à 7 à l'aide d'une solution d'hydroxyde de tetrabutylammonium (40 % p/v). L'addition de la solution est arrêtée quand l'éluat est complètement neutralisé (pH = 7). On lyophilise.
Après lyophilisation on obtient environ 1,3 g de sel.

Etape b- O-sulfatation

On dissout le sel obtenu à l'étape précédente dans 80 ml de formamide, on ajoute 5,6 g de complexe trioxyde de soufre-pyridine (commercialisé par Aldrich® sous la réf. S755-6). On laisse réagir 6 heures à 30°C puis on ajoute 16 ml de NaCl 2 M. On porte à pH 7 à l'aide d'une solution de NaOH 5 N et on précipite par 2 volumes d'éthanol. On reprend le précipité avec une solution de NaCl 0,5 M, on reprécipite avec 2 volumes d'éthanol, on dialyse contre de l'eau ultra-purifiée et on concentre au Rotavapor.

Etape c- Gel-filtration

La solution concentrée obtenue à l'étape b est fractionnée par gel filtration en utilisant une colonne Sephacryl S 300 HR et en utilisant comme éluant une solution de NaCl 0,5 M.
On recueille les fractions contenant un héparosane-N,O-sulfaté constitué de chaînes ayant des masses moléculaires moyennes de 100 000 à 200 000 Da , comme aussi les fractions contenant un héparosane-N,O-sulfaté constitué de chaînes ayant des masses moléculaires moyennes d'environ 50 000 Da et d'environ 12 000 Da.

A chacune de ces trois fractions, on ajoute 5 volumes d'éthanol, on les soumet à une dialyse contre de l'eau ultra-purifiée et on soumet les solutions obtenues après dialyse, à une lyophilisation.

On obtient ainsi 3 lots d'héparosane-N,O-sulfaté :

- le lot 1A est un héparosane-N,O-sulfaté constitué de chaînes ayant une masse moléculaire moyenne de 150 000 à 200 000 Da. Cet héparosane-N,O-sulfaté est constitué d'au moins 70 % en masse de chaînes de masse moléculaire comprise entre $1{,}0.10^5$ et $5{,}0.10^5$ Da. On isole 0,140 g de cet héparosane-N,O-sulfaté.
- le lot 1B est un héparosane-N,O-sulfaté constitué de chaînes ayant une masse moléculaire moyenne d'environ 50 000 Da. Cet héparosane-N,O-sulfaté est constitué d'au moins 70 % en masse de chaînes de masse moléculaire comprise entre $2{,}0.10^4$ et $1{,}0.10^5$ Da. On isole 0,350 g de cet héparosane-N,O-sulfaté.
- le lot 1C est un héparosane-N,O-sulfaté constitué de chaînes ayant une masse moléculaire moyenne de 12 000 Da. On isole environ 0,100 g de ce dernier produit.

Les caractéristiques des trois lots d'héparosane-N,O-sulfaté décrits dans cet exemple sont rassemblés dans le Tableau VI.

## TABLEAU VI

### Caractéristiques des héparosanes-N,O-sulfatés correspondant aux lots 1A, 1B et 1C

| | Taux d'acide uronique (μmole/mg) | Taux de $NH_2$ (μmole/mg) | Taux de désacétylation | Taux de sulfatation (rapport sulfate /carboxyle) |
|---|---|---|---|---|
| **Lot 1A** | 1,48 | < 0,01 | 80 % | 2,6 |
| **Lot 1B** | 1,45 | < 0,01 | 80 % | 2,6 |
| **Lot 1C** | 1,45 | < 0,01 | 80 % | 2,6 |

Le taux de sulfatation, également appelé rapport sulfate/carboxyle, est le nombre moyen de groupes sulfate, contenus dans une structure disaccharidique, par groupe carboxyle contenu dans cette même structure ; il est mesuré par la méthode conductimétrique de dosage du groupe sulfate et du groupe carboxyle, décrite par B. Casu et al., dans Carbohydrate Research, (1975), 39, 168-176.

**EXEMPLE 2**

**Héparosanes-N,O-sulfatés ; dérivés N-désacétylés à 40 % ayant un taux de sulfatation de 2,1**

Etape a - Formation du sel de tétrabutylammonium :

On utilise comme matière première, un héparosane, préparé selon la Préparation V (lot E) et on procède selon la méthode décrite dans l'Exemple 1, Etape a. Le N-acétylhéparosane utilisé comme matière première pour la préparation d'héparosane a été préparé selon le procédé de la Préparation I.

Etape b - N,O-sulfatation partielle :

On dissout 421 mg du sel obtenu à l'étape précédente dans 35 ml de diméthylformamide et on ajoute 3,71 g

de complexe trioxyde de soufre-pyridine.
On laisse réagir sous agitation pendant 6 h à température ambiante. A un volume du milieu réactionnel on ajoute du chlorure de sodium jusqu'à l'obtention d'une solution de concentration 0,33 M en chlorure de sodium, puis 2 volumes d'éthanol. On laisse se former le précipité. On centrifuge et on élimine le surnageant. Le culot de centrifugation est repris dans une solution de NaCl 0,5 M, qu'on neutralise. On ajoute ensuite 2 volumes d'éthanol. On laisse se former le précipité, on centrifuge et on reprend le culot de centrifugation avec de l'eau ultra-purifiée.

On dialyse contre de l'eau ultra-purifiée et on lyophilise.

Le lyophilisat obtenu présente les caractéristiques suivantes :

* taux de $NH_2$ libre : 0,10 µmole/mg
* taux de sulfatation : 1,9 par unité disaccharidique

<u>Etape c</u> - N-sulfatation totale

On mélange dans un volume de 20 ml d'eau ultra-purifiée par gramme de produit N,O-sulfaté engagé, 1 partie pondérale de produit N,O-sulfaté, 1 partie pondérale de bicarbonate de sodium, 1 partie pondérale du complexe trioxyde de soufre-triméthylamine et on laisse réagir à 55°C sous agitation pendant 20 h. On dilue ensuite le mélange réactionnel (facteur de dilution 10), puis on ajuste la conductivité de la solution obtenue à celle d'une solution de chlorure de sodium 0,5 M.

Ensuite sont réalisées une précipitation par ajout de 2 volumes d'éthanol, une centrifugation suivie d'une reprise des culots de centrifugation par une solution de NaCl 0,5 M, puis une seconde précipitation par ajout de 2 volumes d'éthanol. Après reprise dans de l'eau ultra-purifiée et dialyse contre de l'eau ultra-purifiée, le produit est lyophilisé et séché à 40°C sous vide.

Le lyophilisat obtenu (lot 2) présente les caractéristiques suivantes :

* taux de $NH_2$ libres : 0,02 µmole/mg
* taux de sulfatation : 2,1 par unité disaccharidique

On constate que le taux de $NH_2$ résiduel (0,02 µmole/mg), est faible et inférieur à celui de l'héparosane-N,O-sulfaté obtenu à l'issue de l'étape de la N,O-sulfatation partielle. Cela démontre le caractère total de la réaction de la N-sulfatation.

Le lot 2 contient au moins 70 % en masse, de chaînes de masse moléculaire comprise entre $2{,}0.10^4$ Da et $2{,}5.10^5$ Da.

**<u>EXEMPLE 3</u>**

**<u>Héparosane-N,0-sulfaté ; Dérivé désacétylé à 80 % ayant un taux de sulfatation de 2,6</u>**

Comme matière première, on utilise 3,35 g d'un héparosane-N-sulfaté, préparé selon le procédé décrit dans la Préparation VI (Etapes a et b).

Ce composé a été obtenu à partir de 4 g d'un N-acétylhéparosane préparé selon le procédé décrit dans la Préparation III.

L'héparosane-N-sulfaté utilisé comme matière première est constitué à 70 % de chaînes ayant une masse moléculaire comprise entre 20 000 et 150 000 Da, et présente les caractéristiques suivantes :

* Taux d'acide uronique : 1,5 µmole/mg
* Taux de $NH_2$ libres : 0,10 µmole/mg
* Taux de désacétylation : 80 %

<u>Etape a</u> - Formation du sel de tetrabutylammonium

On dissout l'héparosane-N-sulfaté dans 400 ml d'eau ultra-purifiée et l'on procède comme il est décrit dans l'Exemple 1 (Etape a).

Après lyophilisation, on obtient 6,66 g de sel.

<u>Etape b</u> - O-sulfatation

On dissout 297 mg du sel obtenu à l'étape précédente dans 20 ml de formamide et on ajoute 1 g du complexe

trioxyde de soufre-pyridine, puis l'on procède comme il est décrit dans l'Exemple 1, (Etape b).

On obtient ainsi 260 mg d'un héparosane-N,O-sulfaté.

Les caractéristiques de cet héparosane-N,O-sulfaté (lot 3) sont indiquées dans le Tableau VII.

## TABLEAU VII

### Caractéristiques du produit correspondant au lot 3

| | Taux d'acide uronique (μmole/mg) | Taux de $NH_2$ (μmole/mg) | Taux de désacétylation | Taux de sulfatation (rapport sulfate /carboxyle) |
|---|---|---|---|---|
| **Lot 3** | 1,50 | < 0,02 | 80 % | 2,60 |

La répartition des masses moléculaires des chaînes qui constituent l'héparosane-N,O-sulfaté du lot 3 est déterminée par chromatographie d'exclusion stérique selon la technique décrite dans la Préparation I (Première méthode).

La masse moléculaire correspondant au maximum d'absorption a été évaluée à 80 000-150 000 Da. Le lot 3 contient au moins 70 % en masse de chaînes de masse moléculaire comprise entre $2,5.10^4$ Da et $2,5.10^5$Da, et au moins 60 % en masse, de chaînes de masse moléculaire comprise entre $4,5.10^4$ et $2,5.10^5$ Da.

**<u>UTILISATION DES HEPAROSANES N-SULFATES ET DES HEPAROSANES-N,O-SULFATES DE HAUTE MASSE MOLECULAIRE POUR LA PREPARATION DES HEPAROSANES-N,O-SULFATES DE FAIBLE MASSE MOLECULAIRE</u>**

**<u>EXEMPLE 4</u>**

**<u>Préparation d'un héparosane-N,0-sulfaté de faible masse moléculaire à partir d'un héparosane-N,O-sulfaté de haute masse moléculaire ; Dérivé désacétylé à 80 % ayant un taux de sulfation de 2,5</u>**

<u>Etape a</u>- Formation du sel de tétrabutylammonium

On procède comme il est décrit dans l'Exemple 1, étape a, en utilisant 5 g de l'héparosane N-sulfaté obtenu selon la Préparation VI et les quantités de solvants et de réactifs adéquates.

Après lyophilisation, on obtient environ 9,3 g de sel

<u>Etape b</u>- O-sulfatation

8,10 g du sel de tétrabutylammonium précédemment obtenu sont dissous dans 450 ml de formamide anhydre. La solution est passée sur une colonne de 290 ml, de tamis moléculaire 4 Å. On y ajoute alors 27 g de complexe trioxyde de soufre-pyridine, et on incube 6 heures à 30°C.

On additionne alors une quantité adéquate d'une solution de NaCl 2 M, pour que le milieu réactionnel ait une concentration finale en chlorure de sodium égale à 0,5 M. On neutralise ensuite à l'aide de NaOH 1 N, et on précipite par 2 volumes d'éthanol.

On reprend le précipité avec une solution de NaCl à 0,5 M et on reprécipite avec 2 volumes d'éthanol. Après centrifugation, le culot est repris dans un volume minimum d'eau et dialysé pendant 15 heures. La solution obtenue après dialyse est ensuite soumise à une lyophilisation.

On obtient ainsi 5,8 g de produit.

Etape c- Dépolymérisation par l'acide nitreux (concentration finale en nitrite : 0,035 M)

2 g de produit obtenu à l'étape b sont dissous dans 17 ml d'eau ultra-purifiée à 22°C. La solution est dégazée par mise sous vide pendant une heure. Elle est ensuite agitée vigoureusement sous flux d'azote, le pH est abaissé à 2,5 à l'aide d'HCl 5 N et 0,484 ml de nitrite de sodium à 100 mg/ml sont ajoutés.
Le pH est immédiatement réajusté à 2,5 et le volume est complété à 90 ml à l'aide d'eau ultra pure (concentration finale de la solution réactionnelle en nitrite de sodium égale à 0,035 M).
L'agitation est maintenue 3 heures à température ambiante sous courant d'azote. Le pH est ensuite amené à 7,0 au moyen de soude 5 N et 0,4 g de borohydrure de sodium sont ajoutés. La solution est agitée 15 heures à l'air libre. Le borohydrure de sodium en excès est ensuite détruit par acidification à pH 3,5 à l'aide d'acide acétique à 30 %. La solution réactionnelle est ensuite soumise à une agitation violente pendant 15 minutes à l'air libre et le pH est ajusté à 7 par addition de soude 5 N.
L'héparosane fragmenté est récupéré par précipitation à 4 volumes d'éthanol, centrifugation, lavage à l'éthanol sur entonnoir de büchner fritté, et séchage sous vide à 60°C.
On obtient ainsi 8,7 g de produit.

Etape d- Gel-filtration

1,6 g de produit obtenu à l'étape c sont dissous dans 15 ml de NaCl 0,5 M et gel-filtré sur colonne de 5 cm x 100 cm de Sephacryl S 200 HR (PHARMACIA) préalablement équilibrée avec du NaCl 0,5 M, (débit de 8 ml/min). L'éluat est collecté en 30 fractions de 40 ml. La masse moléculaire des fragments d'héparosane-N,O-sulfaté de chacune des fractions est évaluée par HPLC d'exclusion stérique sur une colonne TSK 2000, selon la méthode décrite dans la Préparation VII.
Les fractions ayant une masse moléculaire moyenne comprise entre environ 5500 et 8000 Da sont regroupées.
Le produit contenu dans ces fractions est récupéré par précipitation à 4 volumes d'éthanol, centrifugation, dialyse et lyophilisation.
Les caractéristiques de cet héparosane-N,O-sulfaté de basse masse moléculaire (lot 4) sont indiquées dans le Tableau VIII.

## TABLEAU VIII

### Caractéristiques du produit correspondant au lot 4

| | Taux d'acide uronique (µmole/mg) | Taux de $NH_2$ (µmole/mg) | Taux de désacétylation | Taux de sulfatation (rapport sulfate /carboxyle) |
|---|---|---|---|---|
| Lot 4 | 1,40 | < 0,02 | 80 % | 2,50 |

La répartition des masses moléculaires des chaînes qui constituent l'héparosane-N,O-sulfaté de basse masse moléculaire (Lot 4) est indiquée dans le Tableau IX. Elle a été évaluée par HPLC d'exclusion stérique comme décrit dans la Préparation VII.
$PM_1$, $PM_2$, $PM_3$, $PM_4$ et $PM_5$, ont les significations données pour le Tableau IV.

## TABLEAU IX

Répartition des masses moléculaires du produit correspondant au lot 4

| $PM_1$ | $PM_2$ | $PM_3$ | $PM_4$ | $PM_5$ |
|---|---|---|---|---|
| 14887 | 9445 | 6912 | 5067 | 2754 |

**EXEMPLE 5**

**Préparation d'un héparosane-N,0-sulfaté de faible masse moléculaire à partir d'un héparosane N-sulfaté de haute masse moléculaire ; Dérivé désacétylé à 80 % ayant un taux de sulfatation de 2,9**

Etape a- Dépolymérisation d'un héparosane N-sulfaté, dérivé N-désacétylé à 80 %, par l'acide nitreux (concentration finale en nitrite : 0,035 M)

9 g d'un héparosane N-sulfaté identique à la Préparation VI sont dissous dans 85 ml d'eau ultra purifiée à 22°C. La solution est dégazée par mise sous vide pendant une heure. Elle est ensuite agitée vigoureusement sous flux d'azote, le pH est abaissé à 2,5 au moyen d'HCl 5 N et 2,17 ml de nitrite de sodium à 100 mg/ml sont ajoutés. Le pH est immédiatement réajusté à 2,5 et le volume est complété à 90 ml à l'aide d'eau ultra pure (concentration finale du milieu réactionnel en nitrite de sodium égale à 0,035 M).

L'agitation est maintenue 3 heures à température ambiante sous courant d'azote. Le pH est ensuite amené à 7,0 au moyen de NaOH 5 M et 1,8 g de borohydrure de sodium sont ajoutés. La solution est agitée 15 heures à l'air libre. Le borohydrure en excès est ensuite détruit par acidification à pH 3,5 à l'aide d'acide acétique à 30 %. La solution réactionnelle est ensuite soumise à une agitation violente pendant 15 minutes à l'air libre. Le pH est ajusté à 7,0 par addition de NaOH 5 N.

L'héparosane N-sulfaté fragmenté est récupéré par précipitation par 4 volumes d'éthanol, centrifugation, lavage à l'éthanol sur entonnoir de büchner fritté, et séchage sous vide à 60°C.

8,7 g de produit sont finalement obtenus.

La répartition des masses moléculaires des chaînes qui constituent l'héparosane N-sulfaté (dérivé N-désacétylé à 80 %) obtenu, de faible masse moléculaire (Lot 5A) a été évaluée par HPLC d'exclusion stérique comme décrit dans la Préparation VII.

Les résultats déduits du profil chromatographique sont indiqués dans le tableau X. $PM_1$, $PM_2$, $PM_3$, $PM_4$ et $PM_5$, ont les significations données pour le Tableau IV.

## TABLEAU X

Répartition des masses moléculaires du produit correspondant au lot 5A

| $PM_1$ | $PM_2$ | $PM_3$ | $PM_4$ | $PM_5$ |
|---|---|---|---|---|
| 14282 | 8999 | 3805 | 2177 | 1536 |

Toutes les chaînes dont l'héparosane N-sulfaté du lot 5A est constitué, contiennent à l'extrémité réductrice une structure de formule (c) dans laquelle G représente un atome d'hydrogène.

Etape b- Gel-filtration

1,6 g de produit obtenu à l'étape précédente sont dissous dans 15 ml de NaCl 0,5 M et gel-filtré sur colonne de 5 cm x 100 cm de Sephacryl S 200 HR (PHARMACIA) préalablement équilibrée avec du NaCl 0,5 M, avec un débit de 8 ml/min. L'éluat est collecté en 30 fractions de 40 ml. La masse moléculaire des fragments d'héparosane de chacune des fractions est évaluée par HPLC d'exclusion stérique sur une colonne TSK 2000, selon la méthode décrite dans la préparation VII.
Les fractions ayant une masse moléculaire moyenne comprise entre environ 5 000 et 7 000 Da d'une part (lot 5B1), et entre 3 500 à 5 000 Da d'autre part (lot 5B2), sont regroupées.
L'héparosane N-sulfaté et fragmenté contenu dans les lots 5B1 et 5B2 est récupéré par précipitation à 4 volumes d'éthanol, centrifugation, dialyse et lyophilisation.
On obtient finalement 0,5 g correspondant au lot 5B1 et 0,45 g correspondant au lot 5B2.
La distribution moléculaire de ces deux héparosanes est analysée par HPLC d'exclusion stérique, comme décrit dans la Préparation VII et les résultats sont donnés dans le Tableaux XI.
$PM_1$, $PM_2$, $PM_3$, $PM_4$ et $PM_5$, ont les significations données pour le Tableau IV.

## TABLEAU XI

Répartition des masses moléculaires des produits correspondant aux lots 5B1 et 5B2

| | $PM_1$ | $PM_2$ | $PM_3$ | $PM_4$ | $PM_5$ |
|---|---|---|---|---|---|
| Lot 5B1 | 9943 | 8051 | 6357 | 4611 | 2460 |
| Lot 5B2 | 7800 | 6236 | 4088 | 2400 | 1536 |

Etape c- O-sulfatation des fragments d'héparosane N-sulfaté, N-désacétylé de faible masse moléculaire

840 mg du lot 5B1 sont convertis en sel de tetrabutylammonium par passage sur résine Dowex, préalablement acidifiée, et neutralisés par l'hydroxyde de tetrabutylammonium.
Le sel de tétrabutylammonium est récupéré et lyophilisé.

Procédé I : Sulfatation par le complexe trioxyde de soufre-pyridine

420 mg de l'héparosane N-sulfaté N-désacétylé sous forme de sel de tétrabutylammonium précédemment obtenu sont dissous dans 42 ml de formamide anhydre (la masse indiquée correspond à l'héparosane N-sulfaté, N-désacétylé sous forme non salifiée). La solution est passée sur une colonne de 15 ml, de tamis moléculaire 4 Å. On y ajoute alors 1,4 g de complexe trioxyde de soufre-pyridine, et on incube 6 heures à 30°C.
On additionne alors 9 ml d'une solution de NaCl à 2 M, et on neutralise ensuite à l'aide de NaOH 1 N.
L'héparosane-N,O-sulfaté est précipité par 2 volumes d'éthanol, est centrifugé, redissous dans 10 ml de NaCl à 0,5 M et de nouveau reprécipité avec 2 volumes d'éthanol. Après centrifugation, le culot est repris dans un volume minimum d'eau et dialysé pendant 15 heures.
Le produit est finalement lyophilisé.
On obtient 470 mg d'héparosane-N,O-sulfaté (lot 5C1a). Les caractéristiques de ce produit sont identiques à

celles de l'héparosane-N,O-sulfaté (lot 4) décrit dans l'exemple 4.

Procédé II : Sulfatation par l'acide chlorosulfonique

Les 420 mg de l'héparine N-sulfaté, N-désacétylé sous forme de sel de tétrabutylammonium restants sont dissous dans 8 ml de formamide anhydre (la masse indiquée correspond à l'héparosane N-sulfaté, N-désacétylé sous forme non salifiée). La solution est passée sur une colonne de 15 ml de tamis moléculaire 4 Å et récupérée dans un ballon à triple tubulure raccordé à une garde de chlorure de calcium. On ajoute ensuite 346 μl de pyridine anhydre. En agitant le mélange vigoureusement, on coule alors très lentement 256 mg d'acide chlorosulfonique en solution dans 1 ml de dichlorométhane, en veillant à ce que la température du mélange réactionnel n'excède pas 35°C (durée de l'addition : 15 minutes). La solution est ensuite agitée 2 heures à température ambiante. On additionne ensuite 10 ml de NaCl 0,5 M et la solution est neutralisée par la soude 1 N.

L'héparosane-N,O-sulfaté est précipité par 2 volumes d'éthanol, centrifugé et redissous dans 10 ml de NaCl 0,5 M. Par la suite, il est précipité de nouveau par 2 volumes d'éthanol et centrifugé. Le culot est repris dans un volume minimum d'eau et dialysé extensivement pendant 15 heures.

Après lyophilisation, on obtient 430 mg de produit appelé lot 5C1b.

Les caractéristiques de cet héparosane-N,O-sulfaté de faible masse moléculaire sont indiquées dans le Tableau XII.

## TABLEAU XII

### Caractéristiques du produit correspondant au lot 5C1b

| | Taux d'acide uronique (μmole/mg) | Taux de $NH_2$ (μmole/mg) | Taux de désacétylation | Taux de sulfatation (rapport sulfate /carboxyle) |
|---|---|---|---|---|
| **Lot 5C1b** | 1,50 | < 0,02 | 80 % | 2,90 |

La répartition des masses moléculaires des chaînes qui constituent l'héparosane-N,O-sulfaté du lot 5C1b a été évaluée par HPLC d'exclusion stérique selon la méthode décrite dans la Préparation VII et est indiquée dans le Tableau XIII.

$PM_1$, $PM_2$, $PM_3$, $PM_4$ et $PM_5$, ont les significations données pour le Tableau IV.

## TABLEAU XIII

### Répartition des masses moléculaires du produit correspondant au lot 5C1b

| $PM_1$ | $PM_2$ | $PM_3$ | $PM_4$ | $PM_5$ |
|---|---|---|---|---|
| 11881 | 9116 | 7253 | 5513 | 3056 |

Ce produit est constitué à 70 % de chaînes ayant une masse moléculaire comprise entre 8 792 et 5 900 Da.

## Revendications

1. Héparosanes-N,O-sulfatés constitués par des chaînes ou par un mélange de chaînes de masse moléculaire entre $1{,}5.10^4$ et $4{,}0.10^6$ Da, ayant la structure disaccharidique répétée de formule I :

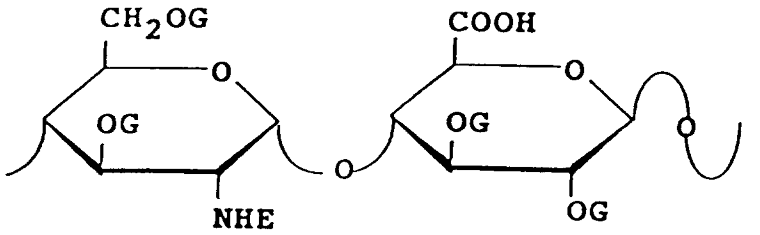

(I)

dans laquelle,

- E représente, dans 0 à 80 % des unités disaccharidiques desdits héparosanes-N,O-sulfatés, un groupe acétyle, et dans les unités disaccharidiques restantes, un groupe sulfate et éventuellement un atome d,hydrogène,
- G représente un atome d'hydrogène et un groupe sulfate,
  et ayant un degré de sulfatation exprimé comme rapport sulfate/carboxyle de 1,5 à 3,0,
  à l'exclusion des héparosanes N,O-sulfatés constitués par des chaînes ayant une masse moléculaire de $1{,}5.10^4$ Da,
  et les sels pharmaceutiquement acceptables desdits héparosanes-N,O-sulfatés.

2. Héparosanes-N,O-sulfatés selon la revendication 1, caractérisés en ce que E représente un groupe acétyle et un groupe sulfate.

3. Héparosanes-N,O-sulfatés selon la revendication 1, constitués par des chaînes ou par un mélange de chaînes de masse moléculaire entre $1{,}5.10^4$ et $4{,}0.10^6$ Da, ayant la structure disaccharidique répétée de formule I :

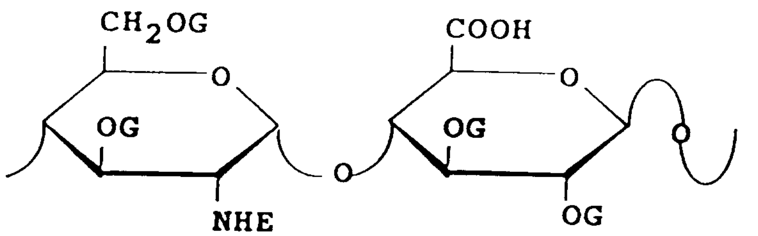

(I)

dans laquelle,

- E représente, dans 0 à 80 % des unités disaccharidiques desdits héparosanes-N,O-sulfatés, un groupe acétyle, et dans les unités disaccharidiques restantes, un groupe sulfate et éventuellement un atome d'hydrogène,
- G représente un atome d'hydrogène et un groupe sulfate,

  le degré de sulfatation, exprimé comme rapport sulfate/carboxyle étant de 1,5 à 3,0,
  les deux extrémités, réductrice et non réductrice des chaînes desdits héparosanes-N,O-sulfatés étant des unités uroniques sulfatées ou non, de la glucosamine sulfatée ou non, ou de la N-acétyl glucosamine, sulfatée ou non,
  et les sels pharmaceutiquement acceptables desdits héparosanes-N,O-sulfatés.

4. Héparosanes-N,O-sulfatés selon l'une quelconque des revendications 1 à 3, caractérisés en ce que E représente dans au plus 60 % des unités disaccharidiques un groupe acétyle.

5. Héparosanes-N,O-sulfatés selon la revendication 1, caractérisés en ce qu'ils contiennent moins de 0,2 μmole/mg de groupe amino libre ($NH_2$).

**6.** Héparosanes-N,O-sulfatés selon l'une quelconque des revendications 1 à 3, constitués par au moins 70 % en masse de chaînes de masse moléculaire entre $1,0.10^5$ et $5,0.10^5$.

**7.** Héparosanes-N,O-sulfatés selon l'une quelconque des revendications 1 à 3, constitués par au moins 70 % en masse de chaînes de masse moléculaire entre $2,5.10^4$ Da et $2,5.10^5$ Da.

**8.** Héparosanes-N,O-sulfatés selon l'une quelconque des revendications 1 à 3, constitués par au moins 70 % en masse de chaînes de masse moléculaire entre $2,0.10^4$ Da et $1,0.10^5$ Da.

**9.** Composition d'héparosane-N,O-sulfaté caractérisée en ce qu'elle contient au moins 70 % en masse d'un héparosane-N,O-sulfaté selon l'une quelconque des revendications 1 à 8.

**10.** Procédé de préparation d'une composition contenant 70 % à 100 % d'un héparosane-N,O-sulfaté selon la revendication 1, caractérisé en ce qu'il comprend la séquence des étapes suivantes :

- <u>étape a</u> : culture d'une souche d'<u>Escherichia coli</u> (K5),

- <u>étape b</u> : isolement et purification - précédé ou non d'une purification préliminaire - du N-acétylhéparosane formé pour obtenir une composition contenant 70 % à 100 % d'un N-acétylhéparosane de haute masse moléculaire constitué par un mélange de chaînes de masse moléculaire entre $1,0.10^4$ et $2,0.10^6$ Da, caractérisées par une structure disaccharidique répétée de formule II :

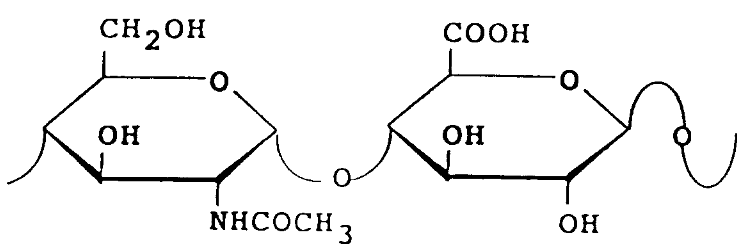

(II)

- <u>étape c</u> : désacétylation partielle de cette composition de N-acétylhéparosane pour obtenir une composition contenant 70 % à 100 % d'un héparosane constitué par un mélange de chaînes de masse moléculaire entre $1,0.10^4$ et $2,0.10^6$ Da, caractérisées par une structure disaccaridique répétée de formule III :

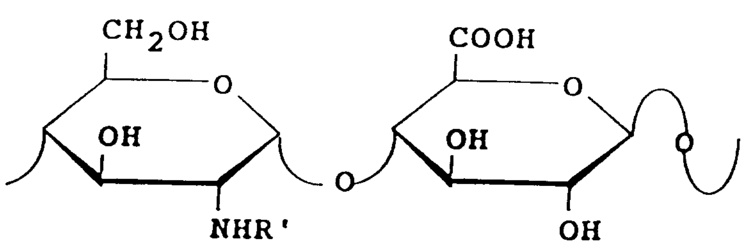

(III)

dans laquelle R' représente, dans 0 à 80 % des unités disaccharidiques, un groupe acétyle, et dans les unités disaccharidiques restantes un atome d'hydrogène,

- <u>étape d</u> :

  - <u>soit</u> une N-sulfatation totale ou partielle pour obtenir une composition contenant 70 à 100 % d'un héparosane N-sulfaté constitué par un mélange de chaînes de masse moléculaire entre $1,0.10^4$ et $2,0.10^6$ Da caractérisée par une structure disaccharidique répétée de formule IV :

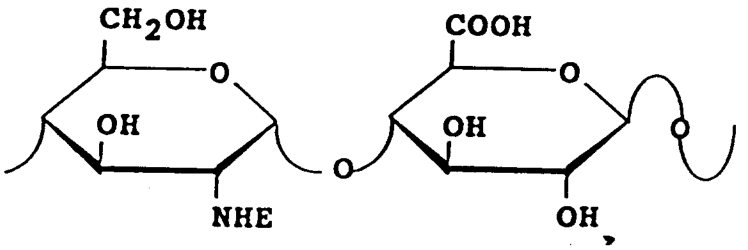

(IV)

dans laquelle E représente dans 0 à 80 % des unités disaccharidiques un groupe acétyle et dans les unités disaccharidiques restantes un groupe sulfate et éventuellement un atome d'hydrogène, cette étape de N-sulfatation partielle ou totale étant suivie d'une étape de O-sulfatation partielle ou totale,

- soit une N,O-sulfatation partielle de cette composition d'héparosane,
- soit une N,O-sulfatation partielle de cette composition d'héparosane suivie d'une étape de N-sulfatation totale,

et comporte éventuellement une ou plusieurs étapes de fractionnement des masses moléculaires effectuées à la fin des étapes a, b, c ou d.

**11.** Procédé selon la revendication 10 caractérisé en ce que la souche d'Escherichia coli (K5) est la souche SEBR 3282 (déposée auprès de la CNCM de l'Institut Pasteur, Paris, France, sous le N° I-1013).

**12.** Procédé selon la revendication 10 caractérisé en ce que la culture de la souche d'Escherichia coli (K5) est effectuée dans un milieu de culture riche en glycérol.

**13.** Procédé selon la revendication 10, caractérisé en ce que l'isolement et la purification du N-acétylhéparosane, comprennent au moins une étape de précipitation alcoolique, et au moins une étape de chromatographie d'échange d'ions.

**14.** Procédé selon la revendication 10 caractérisé en ce que l'isolement et la purification du N-acétylhéparosane sont réalisés à l'aide d'un procédé comportant la séquence des étapes suivantes :

- étape $a_1$ : Précipitation par l'éthanol,
- étape $b_1$ : Dialyse,
- étape $c_1$ : Précipitation par l'éthanol, puis déshydratation et séchage,
- étape $d_1$ : Purification par chromatographie d'échange d'anions,
- étape $e_1$ : Précipitation par l'éthanol de l'éluat obtenu par l'étape $d_1$, déshydratation, séchage et broyage,

dans lequel une des étapes $a_1$ ou $c_1$ peut être supprimée.

**15.** Procédé selon la revendication 10 caractérisé en ce que l'isolement et la purification du N-acétylhéparosane sont réalisés à l'aide d'un procédé comportant la séquence des étapes suivantes :

- étape $a'_1$ : Dialyse,
- étape $b'_1$ : Purification en milieu acide, élimination des impuretés insolubles dans des solutions aqueuses de pH 3.5 et pH 1,8,
- étape $c'_1$ : Précipitation par l'éthanol, puis déshydratation et séchage,
- étape $d'_1$ : Hydrolyse alcaline et dialyse,
- étape $e'_1$ : Purification par chromatographie d'échange d'anions,
- étape $f'_1$ : Purification par chromatographie d'exclusion.

**16.** Procédé selon la revendication 10, caractérisé en ce que le N-acétylhéparosane obtenu de la culture d'une souche d'Escherichia coli (K5) est soumis avant l'isolement et la purification à une purification préliminaire, réalisée à l'aide d'un procédé comportant la séquence des étapes suivantes :

- étape a"$_1$ : Centrifugation de la suspension obtenue à la fin de la culture,
- étape b"$_1$ : Mise en contact du surnageant avec une solution alcaline,
- étape c"$_1$ : Préfiltration,
- étape d"$_1$ : Concentration sur membrane de seuil de coupure déterminé, et éventuellement,
- étape e"$_1$ : Dialyse,

**17.** Composition pharmaceutique contenant comme principe actif un héparosane-N,O-sulfaté selon l'une quelconque des revendications 1 à 8, en association ou en mélange avec un excipient inerte, pharmaceutiquement acceptable.

**18.** Composition pharmaceutique contenant comme principe actif une composition d'héparosane-N,O-sulfaté selon la revendication 9 en association ou en mélange avec un excipient inerte, pharmaceutiquement acceptable.

**19.** Composition pharmaceutique selon l'une quelconque des revendications 17 et 18 utilisable pour la régulation de la coagulation.

**20.** Utilisation des héparosanes-N,O-sulfatés de haute masse moléculaire de formule I, selon la revendication 1, pour la préparation, après fragmentation, des héparosanes-N,O-sulfatés de faible masse moléculaire, inférieure à $1,5.10^4$ Da

**21.** Utilisation des héparosanes N-sulfatés, composés de formule IV, selon la revendication 10, pour la préparation, après fragmentation, des héparosanes-N,O-sulfatés de faible masse moléculaire, inférieure à $1,5.10^4$ Da.

**Claims**

**1.** N,O-sulphated heparosanes consisting of chains or a mixture of chains with a molecular mass of between $1.5 \times 10^4$ and $4.0 \times 10^6$ Da, having the repeated disaccharide structure of formula I:

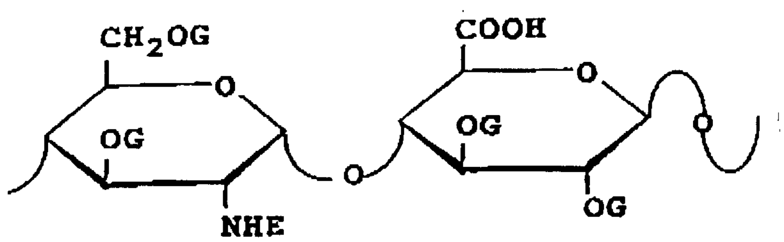

(I)

wherein

- E denotes an acetyl group in 0 to 80% of the disaccharide units of said N,O-sulphated heparosanes, and a sulphate group and optionally a hydrogen atom in the remaining disaccharide units,
- G denotes a hydrogen atom and a sulphate group,

  and having a degree of sulphatation expressed as the ratio of sulphate to carboxyl of 1.5 to 3.0,
  with the exception of N,O-sulphated heparosanes consisting of chains with a molecular mass of $1.5 \times 10^4$ Da,
  and the pharmaceutically acceptable salts of said N,O-sulphated heparosanes.

**2.** N,O-sulphated heparosanes according to claim 1, characterized in that E denotes an acetyl group and a sulphate group.

**3.** N,O-sulphated heparosanes according to claim 1, consisting of chains or a mixture of chains with a molecular mass of between $1.5 \times 10^4$ and $4.0 \times 10^6$ Da, having the repeated disaccharide structure of formula I

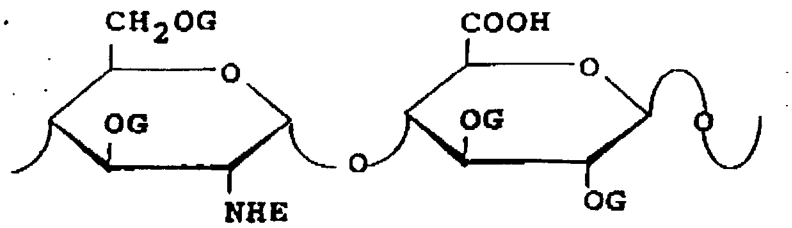

(I)

wherein

- E denotes an acetyl group in 0 to 80% of the disaccharide units of said N,O-sulphated heparosanes, and a sulphate group and optionally a hydrogen atom in the remaining disaccharide units,
- G denotes a hydrogen atom and a sulphate group,
  the degree of sulphatation, expressed as the sulphate/carboxyl ratio, being from 1.5 to 3.0,
  the two ends, the reducing and non-reducing ends of the chains of said N,O-sulphated heparosanes being uronic units which may or may not be sulphated, glucosamine which may or may not be sulphated or N-acetyl glucosamine which may or may not be sulphated,
  and the pharmaceutically acceptable salts of said N,O-sulphated heparosanes.

**4.** N,O-sulphated heparosanes according to any one of claims 1 to 3, characterised in that E denotes an acetyl group in not more than 60% of the disaccharide units.

**5.** N,O-sulphated heparosanes according to claim 1, characterised in that they contain less than 0.2 µmole/mg of free amino group ($NH_2$).

**6.** N,O-sulphated heparosanes according to any one of claims 1 to 3, consisting of at least 70% by mass of chains with a molecular mass of between $1.0x10^5$ and $5.0x10^5$.

**7.** N,O-sulphated heparosanes according to any one of claims 1 to 3, consisting of at least 70% by mass of chains with a molecular mass of between $2.5x10^4$ Da and $2.5x10^5$ Da.

**8.** N,O-sulphated heparosanes according to any one of claims 1 to 3, consisting of at least 70% by mass of chains with a molecular mass of between $2.0x10^4$ Da and $1.0x10^5$ Da.

**9.** Composition of N,O-sulphated heparosane, characterised in that it contains at least 70% by mass of an N,O-sulphated heparosane according to any one of claims 1 to 8.

**10.** Process for preparing a composition containing 70% to 100% of an N,O-sulphated heparosane according to claim 1, characterised in that it comprises the sequence of the following steps:

- step a : cultivating a strain of *Escherichia coli* (K5),

- step b : isolation and purification - which may or may not be preceded by preliminary purification - of the N-acetyl heparosane formed in order to obtain a composition containing 70% to 100% of an N-acetyl heparosane of a high molecular mass consisting of a mixture of chains with a molecular mass of between $1.0x10^4$ and $2.0x10^6$ Da, characterised by a repeated disaccharide structure of formula II:

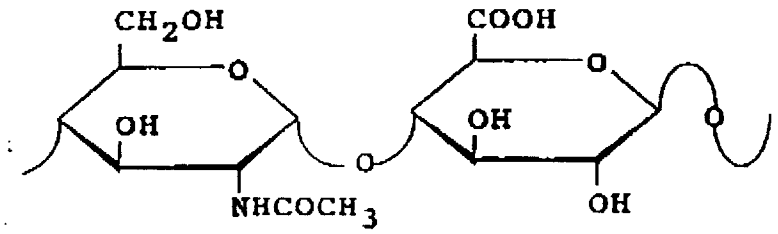

(II)

- step c : partial deacetylation of this N-acetyl heparosane composition in order to obtain a composition containing 70% to 100% of a heparosane consisting of a mixture of chains with a molecular mass of between $1.0 \times 10^4$ and $2.0 \times 10^6$ Da, characterised by a repeated disaccharide structure of formula III:

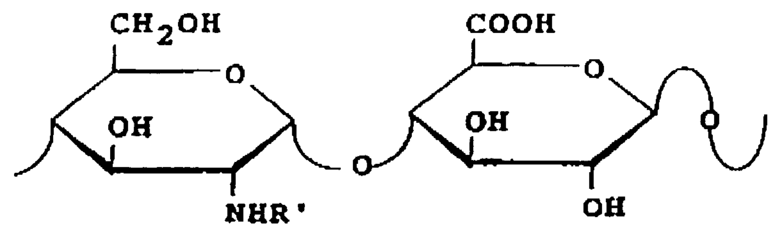

(III)

wherein R' represents an acetyl group in 0 to 80% of the disaccharide units and a hydrogen atom in the remaining disaccharide units,

- step d:

- either total or partial N-sulphatation in order to obtain a composition containing 70 to 100% of an N-sulphated heparosane consisting of a mixture of chains with a molecular weight of between $1.0 \times 10^4$ and $2.0 \times 10^6$ Da, characterised by a repeated disaccharide structure of formula IV:

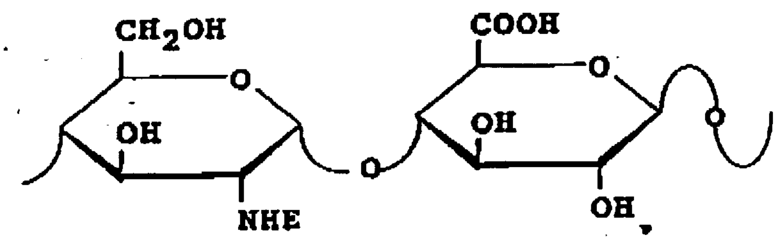

(IV)

wherein E represents an acetyl group in 0 to 80% of the disaccharide units and a sulphate group and optionally a hydrogen atom in the remaining disaccharide units, this step of partial or total N-sulphatation being followed by a step of partial or total O-sulphatation,

- or partial N,O-sulphatation of this heparosane composition,

- or partial N,O-sulphatation of this heparosane composition followed by a step of total N-sulphatation,

and optionally comprises one or more steps of fractionation of the molecular masses carried out at the end of steps a, b, c or d.

11. Process according to claim 10, characterised in that the strain of *Escherichia coli* (K5) is the strain SEBR 3282 (filed at the CNCM of the Institut Pasteur, Paris, France, under No. I-1013).

12. Process according to claim 10, characterised in that the strain of *Escherichia coli* (K5) is cultured in a culture medium rich in glycerol.

13. Process according to claim 10, characterised in that the isolation and purification of the N-acetyl heparosane comprise at least one step of alcoholic precipitation and at least one step of ion exchange chromatography.

14. Process according to claim 10, characterised in that the isolation and purification of the N-acetyl heparosane are carried out using a process comprising the following sequence of steps:

- step $a_1$ : precipitation with ethanol,
- step $b_1$ : dialysis,
- step $c_1$ : precipitation with ethanol, followed by dehydration and drying,
- step $d_1$ : purification by anion exchange chromatography,
- step $e_1$ : precipitation with ethanol of the eluate obtained in step $d_1$, dehydration, drying and grinding,

wherein one of step $a_1$ or $c_1$ may be omitted.

15. Process according to claim 10, characterised in that the isolation and purification of the N-acetyl heparosane are carried out using a process comprising the following sequence of steps:

- step $a'_1$ : dialysis,
- step $b'_1$ : purification in an acidic medium, elimination of the impurities which are insoluble in aqueous solutions of pH 3.5 and pH 1.8,
- step $c'_1$ : precipitation with ethanol followed by dehydration and drying,
- step $d'_1$ : alkaline hydrolysis and dialysis,
- step $e'_1$ : purification by anion exchange chromatography,
- step $f'_1$ : purification by exclusion chromatography.

16. Process according to claim 10, characterised in that the N-acetyl heparosane obtained from culturing a strain of *Escherichia coli* (K5) is subjected, before isolation and purification to a preliminary purification which is carried out using a process comprising the following sequence of steps:

- step $a''_1$ : centrifuging the suspension obtained at the end of culturing,
- step $b''_1$ : bringing the supernatant into contact with an alkaline earth solution,
- step $c''_1$ : prefiltering,
- step $d''_1$ : concentration, on a membrane with a predetermined cutoff threshold, and optionally
- step $e''_1$ : dialysis.

17. Pharmaceutical composition containing as active principle an N,O-sulphated heparosane according to any one of claims 1 to 8, combined or mixed with a pharmaceutically acceptable inert excipient.

18. Pharmaceutical composition containing as active principle a composition of N,O-sulphate heparosane according to claim 9 combined or mixed with a pharmaceutically acceptable inert excipient.

19. Pharmaceutical composition according to any one of claims 17 and 18 which can be used to regulate blood clotting.

20. Use of the N,O-sulphated heparosanes of high molecular mass of formula I according to claim 1 for the preparation, after fragmentation, of N,O-sulphated heparosanes of low molecular mass less than $1.5 \times 10^4$ Da.

21. Use of the N-sulphated heparosanes, compounds of formula IV, according to claim 10 for the preparation, after fragmentation, of the N,O-sulphated heparosanes of low molecular mass less than $1.5 \times 10^4$ Da.

**Patentansprüche**

1. N,O-sulfatierte Heparosane gebildet durch Ketten oder eine Mischung von Ketten mit einer Molekülmasse zwi-

schen $1,5 \cdot 10^4$ und $4,0 \cdot 10^6$ Da, mit der sich wiederholenden Disacharidstruktur der Formel I:

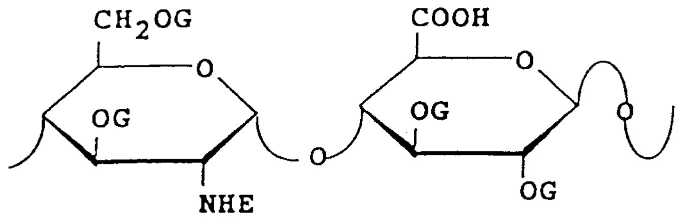

(I)

in der:

- E bei 0 bis 80 % der Disaccharideinheiten der in Rede stehenden N,O-sulfatierten Heparosane eine Acetylgruppe und bei den verbleibenden Disaccharideinheiten eine Sulfatgruppe und gegebenenfalls ein Wasserstoffatom und
- G ein Wasserstoffatom oder eine Sulfatgruppe bedeuten,
  mit einem Sulfatierungsgrad ausgedrückt als Sulfat/Carboxyl-Verhältnis von 1,5 bis 3,0,
  unter Ausschluß von N,O-sulfatierten Heparosanen, die durch Ketten mit einer Molekülmasse von $1,5 \cdot 10^4$ Da gebildet sind,
  und die pharmazeutisch annehmbaren Salze dieser N,O-sulfatierten Heparosane.

**2.** N,O-sulfatierte Heparosane nach Anspruch 1, dadurch gekennzeichnet, daß E eine Acetylgruppe oder eine Sulfatgruppe bedeutet.

**3.** N,O-sulfatierte Heparosane nach Anspruch 1, gebildet durch Ketten oder eine Mischung von Ketten mit einer Molekülmasse zwischen $1,5 \cdot 10^4$ und $4,0 \cdot 10^6$ Da, mit der sich wiederholenden Disaccharidstruktur der Formel I:

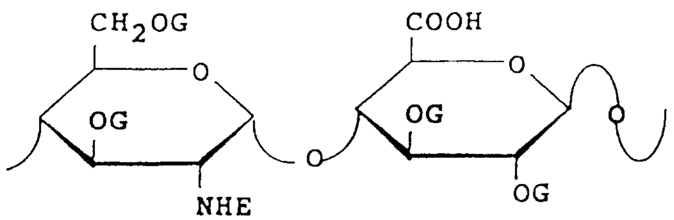

(I)

in der:

- E bei 0 bis 80 % der Disaccharideinheiten der in Rede stehenden N,O-sulfatierten Heparosane eine Acetylgruppe und bei den verbleibenden Disaccharideinheiten eine Sulfatgruppe und gegebenenfalls ein Wasserstoffatom und
- G ein Wasserstoffatom oder eine Sulfatgruppe bedeuten,
  mit einem Sulfatierungsgrad ausgedrückt als Sulfat/Carboxyl-Verhältnis von 1,5 bis 3,0,
  wobei die beiden reduzierenden und nichtreduzierenden Enden der Ketten der genannten N,O-sulfatierten Heparosane gegebenenfalls sulfatierte Uroneinheiten sind des gegebenenfalls sulfatierten Glucosamins oder des gegebenenfalls sulfatierten N-Acetylglucosamins,
  und die pharmazeutisch annehmbaren Salze dieser N,O-sulfatierten Heparosane.

**4.** N,O-sulfatierte Heparosane nach irgendeinem der Ansprüche 1 bis 3, dadurch gekennzeichnet, daß E bei mehr als 60 % der Disaccharideinheiten eine Acetylgruppe bedeutet.

**5.** N,O-sulfatierte Heparosane nach Anspruch 1, dadurch gekennzeichnet, daß sie weniger als 0,2 µMol/mg freie Aminogruppen ($NH_2$) aufweisen.

**6.** N,O-sulfatierte Heparosane nach irgendeinem der Ansprüche 1 bis 3, gebildet aus mindestens 70 Massen-% von Ketten mit einer Molekülmasse zwischen $1,0 \cdot 10^5$ und $5,0 \cdot 10^5$.

**7.** N,O-sulfatierte Heparosane nach irgendeinem der Ansprüche 1 bis 3, gebildet aus mindestens 70 Massen-% Ketten mit einer Molekülmasse zwischen $2{,}5 \cdot 10^4$ Da und $2{,}5 \cdot 10^5$ Da.

**8.** N,O-sulfatierte Heparosane nach irgendeinem der Ansprüche 1 bis 3, gebildet aus mindestens 70 Massen-% Ketten mit einer Molekülmasse zwischen $2{,}0 \cdot 10^4$ Da und $1{,}0 \cdot 10^5$ Da.

**9.** N,O-sulfatierte Heparosan-Zubereitung, dadurch gekennzeichnet, daß sie mindestens 70 Massen-% eines N,O-sulfatierten Heparosans nach irgendeinem der Ansprüche 1 bis 8 enthält.

**10.** Verfahren zur Herstellung einer Zubereitung, die 70 % bis 100 % eines N,O-sulfatierten Heparosans nach Anspruch 1 enthält, dadurch gekennzeichnet, daß es die Folge der nachstehenden Stufen umfaßt:

- Stufe a: Züchtung eines Stammes von Escherichia coli (K5),
- Stufe b: Isolierung und Reinigung - gegebenenfalls nach einer vorläufigen Reinigung - des gebildeten N-Acetylheparosans zur Bildung einer Zubereitung, die 70 % bis 100 % eines N-Acetylheparosans mit hoher Molekülmasse enthält, welches aus einer Mischung von Ketten mit Molekülmassen zwischen $1{,}0 \cdot 10^4$ und $2{,}0 \cdot 10^6$ Da gebildet ist, gekennzeichnet durch eine sich wiederholende Disaccharidstruktur der Formel II:

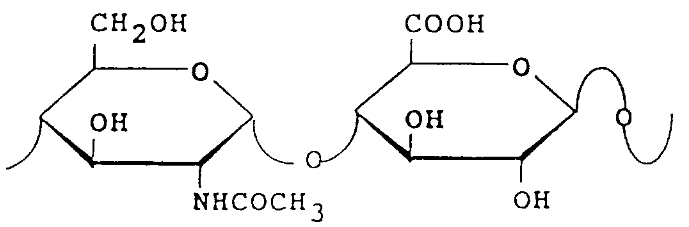

(II)

- Stufe c: teilweise Desacetylierung dieser N-Acetylheparosan-Zubereitung zur Bildung einer Zubereitung, die 70 % bis 100 % eines Heparosans enthält, welches aus einer Mischung von Ketten mit einer Molekülmasse zwischen $1{,}0 \cdot 10^4$ und $2{,}0 \cdot 10^6$ Da gebildet ist, gekennzeichnet durch eine sich wiederholende Disaccharidstruktur der Formel III:

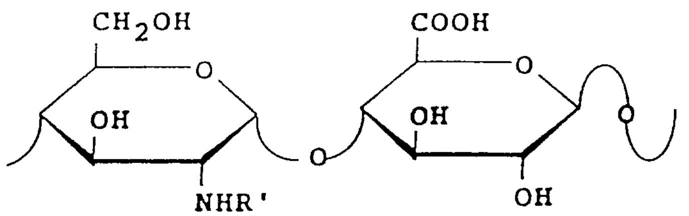

(III)

in der R' bei 0 bis 80 % der Disaccharideinheiten eine Acetylgruppe und bei den verbleibenden Disaccharideinheiten ein Wasserstoffatom bedeutet,

- Stufe d:

  - entweder vollständige oder teilweise N-Sulfatierung zur Bildung einer Zubereitung, die 70 bis 100 % eines N-sulfatierten Heparosans enthält, welches durch eine Mischung aus Kettenmit einer Molekülmasse zwischen $1{,}0 \cdot 10^4$ und $2{,}0 \cdot 10^6$ Da gebildet ist, gekennzeichnet durch eine sich wiederholende Disaccharidstruktur der Formel IV:

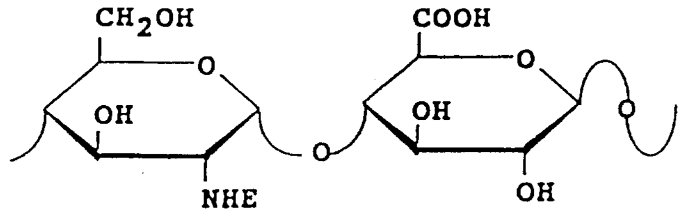

(IV)

in der E bei 0 bis 80 % der Disaccharideinheiten eine Acetylgruppe und bei den verbleibenden Disaccharideinheiten eine Sulfatgruppe und gegebenenfalls ein Wasserstoffatom bedeutet,
welche Stufe der teilweisen oder vollständigen N-Sulfatierung gefolgt wird von einer Stufe einer teilweisen oder vollständigen O-Sulfatierung,

- oder eine teilweise N,O-Sulfatierung dieser Heparosan-Zubereitung,
- oder eine teilweise N,O-Sulfatierung dieser Heparosan-Zubereitung gefolgt von einer Stufe der vollständigen N-Sulfatierung,

welches gegebenenfalls eine oder mehrere Stufen der Fraktionierung der Molekülmassen am Ende der Stufen a, b, c oder d umfaßt.

**11.** Verfahren nach Anspruch 10, dadurch gekennzeichnet, daß der Stamm von Escherichia coli (K5) der Stamm SEBR 3282 (hinterlegt bei der CNCM des Institut Pasteur, Paris, Frankreich unter der Nr. I-1013) ist.

**12.** Verfahren nach Anspruch 10, dadurch gekennzeichnet, daß das Züchten des Stammes Escherichia coli (K5) in einem an Glycerol reichen Kulturmedium erfolgt.

**13.** Verfahren nach Anspruch 10, dadurch gekennzeichnet, daß die Isolierung und die Reinigung des N-Acetylheparosans mindestens eine Stufe der Ausfällung mit Alkohol und mindestens eine Stufe einer Ionenaustauschchromatographie umfaßt.

**14.** Verfahren nach Anspruch 10, dadurch gekennzeichnet, daß die Isolierung und Reinigung des N-Acetylheparosans mit Hilfe eines Verfahrens erfolgt, welches die Folge der nachstehenden Stufen umfaßt:

- Stufe $a_1$: Ausfällung mit Ethanol,
- Stufe $b_1$: Dialyse,
- Stufe $c_1$: Ausfällung mit Ethanol und dann Entwässerung und Trocknung,
- Stufe $d_1$: Reinigung durch Anionenaustauschchromatographie,
- Stufe $e_1$: Ausfällung des in der Stufe $d_1$ erhaltenen Eluats mit Ethanol, Entwässerung, Trocknung und Verreiben,

wobei eine der Stufen $a_1$ oder $c_1$ auch nicht durchgeführt werden kann.

**15.** Verfahren nach Anspruch 10, dadurch gekennzeichnet. daß die Isolierung und Reinigung des N-Acetylheparosans mit Hilfe eines Verfahrens erfolgt, welches die Folge der nachstehenden Stufen umfaßt:

- Stufe $a'_1$: Dialyse,
- Stufe $b'_1$: Reinigung in saurem Medium, Entfernung der in wäßrigen Lösungen mit einem pH-Wert von 3,5 und einem pH-Wert von 1,8 unlöslichen Verunreinigungen,
- Stufe $c'_1$: Ausfällung mit Ethanol und dann Entwässerung und Trocknung,
- Stufe $d'_1$: alkalische Hydrolyse und Dialyse,
- Stufe $e'_1$: Reinigung durch Anionenaustauschchromatographie,
- Stufe $f'_1$: Reinigung durch Ausschlußchromatographie.

**16.** Verfahren nach Anspruch 10, dadurch gekennzeichnet, daß das beim Züchten des Stammes Escherichia coli (K5) erhaltene N-Acetylheparosan vor der Isolierung und der Reinigung einer vorläufigen Reinigung unterworfen wird, welche mit Hilfe eines Verfahrens durchgeführt wird, das die Folge der nachstehenden Stufen umfaßt:

- Stufe a"$_1$: Zentrifugieren der am Ende des Züchtungsvorgangs erhaltenen Suspension,
- Stufe b"$_1$: Inkontaktbringen der überstehenden Flüssigkeit mit einer alkalischen Lösung,
- Stufe c"$_1$: Vorfiltration,
- Stufe d"$_1$: Konzentration auf einer Membran mit einer vorher bestimmten Abtrennschwelle und gegebenenfalls
- Stufe e"$_1$: Dialyse.

**17.** Pharmazeutische Zubereitung enthaltend als Wirkstoff ein N,O-sulfatiertes Heparosan nach irgendeinem der Ansprüche 1 bis 8 in Kombination oder in Mischung mit einem inerten, pharmazeutisch annehmbaren Trägermaterial.

**18.** Pharmazeutische Zubereitung enthaltend als Wirkstoff eine N,O-sulfatierte Heparosan-Zubereitung nach Anspruch 9 in Kombination oder in Mischung mit einem inerten, pharmazeutisch annehmbaren Trägermaterial.

**19.** Pharmazeutische Zubereitung nach irgendeinem der Ansprüche 17 und 18 zur Steuerung der Koagulation.

**20.** Verwendung von N,O-sulfatierten Heparosanen hoher Molekülmasse der Formel I nach Anspruch 1 für die Herstellung durch Fragmentation von N,O-sulfatierten Heparosanen niedriger Molekülmasse von weniger als $1,5 \cdot 10^4$ Da.

**21.** Verwendung der N-sulfatierten Heparosane der Formel IV nach Anspruch 10 für die Herstellung durch Fragmentierung von N,O-sulfatierten Heparosanen niedriger Molekülmasse von weniger als $1,5 \cdot 10^4$ Da.